# FASCICULE DE BREVET EUROPEEN

(11) **EP 1 071 767 B1**
(45) Date de publication et mention de la délivrance du brevet: **11.07.2007**
(21) Numéro de dépôt: 99913384.6
(22) Date de dépôt: 12.04.1999
(51) Int. Cl.: C12N 15/12, C07K 14/435, C12N 15/82, A61K 38/17, C12P 21/02, C12N 15/62, C12N 15/81

(54) **GENE CODANT POUR L'HELIOMICINE ET SON UTILISATION**
HELIOMYCIN-KODIERENDES GEN UND SEINE VERWENDUNG
GENE CODING FOR HELIOMICINE AND USE THEREOF

(30) Priorité: 15.04.1998 FR 9804933
(43) Date de publication de la demande: 31.01.2001
(73) Titulaire: Novozymes A/S, 2880 Bagsvaerd (DK)
(72) Inventeur: LAMBERTY, Mireille, F-67100 Strasbourg (FR); BULET, Philippe, F-67550 Vendenheim (FR); BROOKHART, Gary, Lee, Durham, NC 27713 (US); HOFMANN, Jules, F-67000 Strasbourg (FR)
(86) Numéro de dépôt international: PCT/FR1999/000843
(87) Numéro de publication internationale: WO 1999/053053

(56) Documents cités:
- EP-A- 0 307 841
- EP-A- 0 607 080
- WO-A-90/11770
- WO-A-97/30082
- DE-A- 2 212 854
- FR-A- 2 695 392
- FR-A- 2 725 992
- CHUNG K T ET AL: "Antibacterial factors in immune hemolymph from heliothis virescens larvae" ABSTRACTS OF THE GENERAL MEETING OF THE AMERICAN SOCIETY FOR MICROBIOLOGY, vol. 96, no. 0, 19 mai 1996 (1996-05-19), page 275 XP002089180 WASHINGTON US
- HOFFMANN J A ET AL.: "Insect defensins: inducible antibacterial peptides" IMMUNOLOGY TODAY., vol. 13, no. 10, 1992, pages 411-415, XP002089181 CAMBRIDGE GB
- LAMBERTY M ET AL.: "Insect immunity - Isolation from the lepidopteran Heliothis virescens of a novel insect defensin with potent antifungal activity" JOURNAL OF BIOLOGICAL CHEMISTRY., vol. 274, no. 14, 2 avril 1999 (1999-04-02), pages 9320-9326, XP002112857 BALTIMORE, US ISSN: 0021-9258

## Description

La présente invention a pour objet un nouveau peptide riche en cystéines appelé héliomicine, son utilisation a titre de médicament et les compositions le comprenant, une séquence d'ADN codant pour ce peptide, un vecteur la contenant pour la transformation d'un organisme hôte et le procédé de transformation dudit organisme.

L'invention concerne plus particulièrement la transformation des cellules végétales et des plantes, l'héliomicine produite par les plantes transformées leur conférant une résistance aux maladies, en particulier d'origine fongique.

Il existe aujourd'hui un besoin grandissant de rendre les plantes résistantes contre les maladies notamment fongiques afin de diminuer, voire d'éviter, d'avoir recours à des traitements avec des produits de protection antifongiques, en vue de protéger l'environnement. Un moyen d'augmenter cette résistance aux maladies consiste à transformer les plantes de manière qu'elles produisent des substances à même d'assurer leur défense contre ces maladies.

Dans le domaine de la santé humaine, il existe des infections fongiques opportunistes pour lesquelles aucun traitement réellement efficace n'est disponible à l'heure actuelle. En particulier, c'est le cas de certaines mycoses invasives graves qui touchent des patients hospitalisés dont le système immunitaire est déprimé à la suite d'une transplantation, d'une chimiothérapie ou de l'infection par le VIH. En comparaison de l'arsenal des agents antibactériens, la panoplie actuelle des agents antifongiques est très limitée. Il existe donc un besoin réel de caractériser et de développer de nouvelles classes de substances antifongiques.

On connaît différentes substances d'origine naturelle, en particulier des peptides, présentant des propriétés bactéricides ou fongicides, notamment contre les champignons responsables des maladies des plantes. Toutefois, un premier problème consiste à trouver de telles substances qui pourront non seulement être produites par des plantes transformées, mais encore conserver leurs propriétés bactéricides ou fongicides et les conférer aux dites plantes. Au sens de la présente invention, on entend par bactéricide ou fongicide tant les propriétés bactéricides ou fongicides proprement dites que les propriétés bactériostatiques ou fongistatiques.

On connaît également des peptides riches en cystéines présentant des activités bactéricides ou bactériostatiques, mais qui ne présentent pas d'activité fongicide ou fongistatique. Un autre problème consiste à trouver un peptide riche en cystéines présentant une forte activité fongicide ou fongistatique par rapport aux peptides de l'état de la technique.
FR 2695392 enseigne l'existence de défensines isolées de larves d'insectes, à activité antibactérienne. Aucune activité antifongique n'est démontrée, ni même envisagée dans la description.
WO 90/11770 décrit des défensines isolées de mammifères, à activité antifongique et antibactérienne. Ces défensines sont ainsi nettement différentes structurellement des molécules selon l'invention.
En effet, Hoffmann et al (Immunol. Today, 1992, 13, 10, 411-5) est une revue faisant le point sur l'état de l'art, et indique clairement que les défensines d'insectes possèdent des activités antibactériennes, essentiellement contre des bactéries Gram-positives, à un degré moindre Gram-négatives, en diffèrant fortement des défensines de mammifères, en particulier d'un point de vue structural, ancêtre commun, ou localisation. Ce document indique également qu'il n'a été possible de détecter aucune activité antifongique pour les défensines d'insectes, contrairement aux défensines de mammifères.

L'héliomicine est un peptide isolé à partir de l'hémolymphe du lépidoptère *Heliothis virescens* qui présente une activité fongicide contre les champignons responsables des maladies des plantes et les champignons de la pathologie humaine et animale. Après avoir d'abord synthétisé le gène de l'héliomicine, on a également trouvé qu'il pouvait être inséré dans un organisme hôte, comme une levure ou une plante, pour exprimer l'héliomicine et soit produire de l'héliomicine purifiée ou non, soit conférer au dit organisme hôte des propriétés de résistance aux maladies fongiques, apportant une solution particulièrement avantageuse aux problèmes énoncés ci-dessus.

L'invention- a donc d'abord pour objet l'héliomicine, son utilisation a titre de médicament ou en agrochimie pour la protection des plantes, les compositions le comprenant, un fragment d'acide nucléique codant pour l'héliomicine, un gène chimère comprenant ledit fragment codant pour l'héliomicine ainsi que des éléments de régulation en position 5' et 3' hétérologues pouvant fonctionner dans un organisme hôte, en particulier dans les levures ou les plantes et un vecteur pour la transformation des organismes hôtes contenant ce gène chimère, et l'organisme hôte transformé. Elle concerne aussi une cellule végétale transformée contenant au moins un fragment d'acide nucléique codant pour l'héliomicine et une plante résistante aux maladies contenant la dite cellule, en particulier régénérée à partir de cette cellule. Elle concerne enfin un procédé de transformation des plantes pour les rendre résistantes aux maladies dans lequel on insère un gène codant pour l'héliomicine au moyen d'un vecteur approprié. Elle concerne enfin un procédé de préparation de l'héliomicine par des organismes hôtes transformés.

Par héliomicine, on entend selon l'invention tout peptide comprenant essentiellement la séquence peptidique de formule (I) ci-dessous,

Xaa-Cys-Xab-Cys-Xac-Cys-Xad-Cys-Xae-Cys-Xaf-Cys-Xag (I)

dans laquelle:
Xaa est -NH₂ ou un reste peptidique comprenant de 1 à 10 acides aminés, de préférence de 1 à 6 acides aminés, comprenant au moins un acide aminé basique, représentant la séquence peptidique suivante Xaa'-Gly-Xaa"- dans laquelle Xaa' représente NH₂ ou un reste peptidique comprenant 1 à 9 acides aminés, de préférence 1 à 5 acides aminés, et Xaa" représente un reste peptidique comprenant au moins un acide aminé, choisi de préférence parmi Leu, Ilc, Val, Pro, Ser ou Thr
Xab est un reste peptidique comprenant de 1 à 10 acides aminé, de préférence 10,
Xac est un reste peptidique de 3 acides aminés comprenant au moins un acide aminé acide,
Xad représente la séquence peptidique suivante -Lys-Arg-Arg-Gly-Tyr-Lys-Gly-Gly-His-
Xae représente la séquence peptidique suivante -Gly-Xae'-Asn-, dans laquelle Xae' représente un reste peptidique comprenant 5 acides aminés,
Xaf est un tryptophane, et
Xag est -OH ou un reste peptidique comprenant de 1 à 2 acides aminés.

Par acides aminés basiques, on entend plus particulièrement selon l'invention les acides aminés choisis parmi la lysine, l'arginine ou l'homoarginine.

Selon un autre mode préférentiel de réalisation de l'invention, Xac comprend exactement un acide aminé acide.

De manière avantageuse, Xac représente la séquence peptidique suivante -Asn-Xac'-Xac"-, dans laquelle Xac' représente un reste peptidique de 1 acide aminé, et Xac" représente un reste peptidique de 1 acide aminé acide.

Par acide aminé acide on entend selon l'invention tout acide aminé comprenant sur une chaîne latérale une fonction acide organique, plus particulièrement acide carboxylique, de préférence choisi parmi l'acide glutamique (Glu) ou l'acide aspartique (Asp).

De manière préférentielle, Xac représente la séquence peptidique suivante -Asn-Gly-Glu- ou -Ala-Ala-Glu-.

De manière avantageuse,
Xaa représente la séquence peptidique suivante Xaa'-Gly-Xaa"- dans laquelle Xaa' représente NH₂ ou un reste peptidique comprenant 1 à 9 acides aminés, de préférence 1 à 5 acides aminés, et Xaa" représente un reste peptidique comprenant au moins un acide aminé, choisi de préférence parmi Leu, Ile, Val, Pro, Ser ou Thr, et/ou
Xab représente la séquence peptidique suivante -Val-Xab'-Asp-, dans laquelle Xab' représente un reste peptidique comprenant de 0 à 8 acides aminés, de préférence 8, et/ou
Xae représente la séquence peptidique suivante -Gly-Xae'-Asn-, dans laquelle Xae' représente un reste peptidique comprenant 5 acides aminés, et/ou
Xaf est un tryptophane et/ou
Xag représente la séquence peptidique suivante -Glu-Xag' dans laquelle Xag' représente OH ou un reste comprenant 1 acide aminé.

Selon un mode de réalisation plus préférentiel de l'invention, Xaa représente la séquence peptidique suivante NH₂-Asp-Lys-Leu-Ile-Gly-Ser- ou NH₂-Ala-Ala-Ala-Ala-Gly-Ser-, et/ou Xab représente la séquence peptidique suivante -Val-Trp-Gly-Ala-Val-Asn-Tyr-Thr-Ser-Asp-, et/ou Xae représente la séquence peptidique suivante -Gly-Ser-Phc-Ala-Asn-Val-Asn-, et/ou Xaf représente l'acide aminé suivant -Trp-, et/ou Xag représente la séquence peptidique suivante -Glu-Thr-OH ou -Arg-Thr-OH.

Selon un mode de réalisation plus préférentiel de l'invention, l'héliomicine est le peptide représenté avec sa séquence codante par l'identificateur de séquence n° 2 (SEQ ID NO 2). La même séquence est décrite, correspondant aux acides aminés 6 à 49 de l'identificateur de séquence n° 1 (SEQ ID NO 1) avec une séquence codante différente.

Le résidu NH2 terminal peut présenter une modification post-traductionnelle, par exemple une acétylation, de même que le résidu C-terminal peut présenter une modification post-traductionnelle, par exemple une amidation.

Par séquence peptidique comprenant essentiellement la séquence peptidique de formule générale (I), on entend non seulement les séquences définies ci-dessus, mais également de telles séquences comprenant à l'une ou l'autre de leurs extrémités, ou les deux, des résidus peptidiques nécessaires à leur expression et ciblage dans un organisme hôte. Par organisme hôte on entend tout organisme comprenand au moins une cellule, qu'il s'agisse de microorganismes, en particulier une levure ou une bactérie, ou encore de cellules végétales ou encore d'organismes supérieurs comme les plantes.

Il s'agit en particulier d'un peptide de fusion «peptide-héliomicine», dont la coupure par les systèmes enzymatiques de l'organisme hôte permet la libération de l'héliomicine, l'héliomicine étant définie ci-dessus. Le peptide fusionné à l'héliomicine peut être un peptide signal ou un peptide de transit qui permet de contrôler et d'orienter la production de l'héliomicine de manière spécifique dans une partie de l'organisme hôte, comme par exemple le cytoplasme, la membrane cellulaire, ou dans le cas des plantes dans un type particulier de compartiments cellulaires ou de tissus ou dans la matrice extracellulaire.

Selon un mode de réalisation, le peptide de transit peut être un signal d'adressage chloroplastique ou mitochondrial, lequel est ensuite clivé dans les chloroplastes ou les mitochondries.

Selon un autre mode de réalisation de l'invention, le peptide signal peut être un signal N-terminal ou « prépeptide », éventuellement en association avec un signal responsable de la rétention de la protéine dans le réticulum endoplasmique, ou un peptide d'adressage vacuolaire ou « propeptide ». Le réticulum endoplasmique est le lieu où sont pris en charge par la « machinerie cellulaire » des opérations de maturation de la protéine produite, comme par exemple le clivage du peptide signal.

Les peptides de transit peuvent être soit simples, soit doubles, et dans ce cas éventuellement séparés par une séquence intermédiaire, c'est à dire comprenant, dans le sens de la transcription, une séquence codant pour un peptide de transit d'un gène végétal codant pour une enzyme à localisation plastidiale, une partie de séquence de la partie mature N-terminale d'un gène végétal codant pour une enzyme à localisation plastidiale, puis une séquence codant pour un second peptide de transit d'un gène végétal codant pour une enzyme à localisation plastidiale, tels que décrit dans la demande EP 0 508 909.

Comme peptide de transit utile selon l'invention, on peut citer en particulier le peptide signal du gène PR-1α du tabac décrit par Comelissen & coll., représenté avec sa séquence codante par l'identificateur de séquence n° 2, en particulier lorsque l'héliomicine est produite par des cellules végétales ou des plantes, ou du précurseur du facteur Mat α1 lorsque l'héliomicine est produite dans des levures.

Le peptide de fusion « MFα1/héliomicine » avec les cinq résidus du propeptide du facteur MFα1 (Ser-Leu-Asp-Lys-Arg), situés en position N-terminale et sa séquence codante sont partie de la présente invention, en particulier décrits par l'identificateur de séquence n° 1 (SEQ ID NO 1), correspondants aux acides aminés 1 à 49.

Le peptide de fusion « peptide signal PR-1α -héliomicine » et sa séquence codante sont également partie de la présente invention, en particulier décrits par l'identificateur de séquence n° 3 (SEQ ID NO 3).

Le peptide de fusion comprenant le peptide signal du gène PG1 de polygalacturonase de maïs fusioné à l'héliomicine « peptide signal PG1/héliomicine » est représenté avec sa séquence codante par les identificateurs de séquences n° 18 et 20 (SEQ ID NO 18 et SEQ ID NO 20).

Selon un mode préférentiel de réalisation de l'invention, les résidus cystéines du peptide de formule (I) forment au moins un pont disulfure intramoléculaire, de préférence trois ponts disulfure. Selon un mode préférentiel de réalisation de l'invention les ponts disulfure sont établis entre les résidus cystéine 1 et 4, 2 et 5 et 3 et 6.

L'héliomicine est un peptide particulièrement actif contre les champignons et les levures, et peut être a ce titre employé a titre préventif ou curatif pour protéger différents organismes contre des agressions fongiques. La présente invention concerne donc l'héliomicine a titre de médicament. Elle concerne également l'utilisation de l'héliomicine pour le traitement des plantes contre des agressions fongiques, en appliquant l'héliomicine directement sur les dites plantes.

La présente invention concerne également une composition comprenant l'héliomicine et un véhicule approprié. Le véhicule approprié a pour première qualité de ne pas dégrader de manière substantielle l'héliomicine dans la composition, et de ne pas diminuer les propriétés bactéricides et fongicides de l'héliomicine. Cette composition peut être une composition cosmétique et dans ce cas le véhicule approprié est cosmétiquement acceptable (adapté en outre pour une application sur la peau ou les phanères), ou une composition pharmaceutique pour un usage thérapeutique et dans ce cas le véhicule approprié est pharmaceutiquement acceptable approprié pour une administration de l'héliomicine par voie topique, per os ou par injection, ou encore une composition agrochimique et dans ce cas le vehicule approprié est agrochimiquement acceptable, approprié pour une application sur les plantes ou a proximité des plantes, sans les dégrader.

La présente invention concerne également un fragment d'acide nucléique, en particulier d'ADN. naturel ou synthétique, codant pour l'héliomicine définie ci-dessus, y compris pour le peptide de fusion « peptide-héliomicine» défini ci-dessus. Il peut s'agir selon l'invention d'un fragment synthétise ou isolé du lépidoptère *Heliothis,* ou encore un fragment dérivé, adapté pour l'expression de l'héliomicine dans l'organisme hôte où le peptide sera exprimé. Le fragment d'acide nucléique peut être obtenu selon les méthodes standards d'isolation et de purification, ou encore par synthèse selon les techniques usuelles d'hybridations successives d'oligonucléotides synthétiques. Ces techniques sont notamment décrites par Ausubel *et al.*

Selon la présente invention, on entend par « fragment d'acide nucléique » une séquence nucléotidique pouvant être de type ADN ou ARN, de préférence de type ADN, notamment double brin.

Selon un mode de réalisation de l'invention, le fragment d'acide nucléique codant pour l'héliomicine comprend la séquence d'ADN décrite par les bases 16 à 147 de l'identificateur de séquence n° 1 (SEQ ID NO 1), ou par l'identificateur de séquence n° 2 (SEQ ID NO 2), en particulier la partie codante de cette séquence correspondant aux bases 1 à 132, une séquence homologue ou une séquence complémentaire de ladite séquence.

Selon un autre mode de réalisation de l'invention, le fragment d'acide nucléique codant pour le peptide de fusion « peptide-héliomicine » comprend la séquence d'ADN décrite par l'identificateur de séquence n°1 (SEQ ID NO 1) ou celle décrite par l'identificateur de séquence n° 3 (SEQ ID NO 3), en particulier la partie codante correspondant aux bases 3 à 224, ou celle décrite par l'identificateur de séquence n° 18 (SEQ ID NO 18), en particulier la partie codante correspondant aux bases 7 à 205, une séquence homologue ou une séquence complémentaire des dites séquences.

Par « homologue », on entend selon l'invention un fragment d'acide nucléique présentant une ou plusieurs modifications de séquence par rapport à la séquence nucléotidique décrite par les identificateurs de séquences n° 1, 2 ou 3 et codant pour l'héliomicine ou le peptide de fusion « peptide-héliomicine ». Ces modifications peuvent être obtenues selon les techniques usuelles de mutation, ou encore en choisissant les oligonucléotides synthétiques employés dans la préparation de ladite séquence par hybridation. Au regard des multiples combinaisons d'acides nucléiques pouvant conduire à l'expression d'un même acide aminé, les différences entre la séquence de référence décrite par les identificateurs de séquences n° 1, 2 ou 3 et l'homologue correspondant peuvent être importantes, d'autant plus qu'il s'agit de fragments d'ADN de faible taille réalisables par synthèse chimique. De manière avantageuse, le degré d'homologie sera d'au moins 70 % par rapport à la séquence de référence, de préférence d'au moins 80 %, plus préférentiellement d'au moins 90 %. Ces modifications sont généralement neutres, c'est à dire qu'elles n'affectent pas la séquence primaire de l'héliomicine ou du peptide de fusion résultants.

La présente invention concerne également un gène chimère (ou cassette d'expression) comprenant une séquence codante ainsi que des éléments de régulation en position 5' et 3' hétérologues pouvant fonctionner dans un organisme hôte, en particulier les cellules végétales ou les plantes, la séquence codante comprenant au moins un fragment d'ADN codant pour l'héliomicine ou le peptide de fusion « peptide-héliomicine » tel que définis ci-dessus.

Par organisme hôte, on entend tout organisme mono ou pluricellulaire, inférieur ou supérieur, dans lequel le gène chimère selon l'invention peut être introduit, pour la production d'héliomicine. Il s'agit en particulier de bactéries, par exemple *E*. *coli,* de levures, en particulier des genres *Saccharomyces* ou *Kluyveromyces, Pichia,* de champignons, en particulier *Aspergillus,* d'un baculovirus, ou de préférence des cellules végétales et des plantes.

Par "cellule végétale", on entend selon l'invention toute cellule issue d'une plante et pouvant constituer des tissus indifférenciés tels que des cals, des tissus différenciés tels que des embryons, des parties de plantes, des plantes ou des semences.

On entend par "plante" selon l'invention, tout organisme multicellulaire différencié capable de photosynthèse, en particulier monocotylédones ou dicotylédones, plus particulièrement des plantes de culture destinées ou non à l'alimentation animale ou humaine, comme le maïs, le blé, le colza, le soja, le riz, la canne à sucre, la betterave, le tabac, le coton, etc.

Les éléments de régulation nécessaires à l'expression du fragment d'ADN codant pour l'héliomicine sont bien connus de l'homme du métier en fonction de l'organisme hôte. Ils comprennent notamment des séquences promotrices, des activateurs de transcription, des séquences terminatrices, y compris des codons start et stop. Les moyens et méthodes pour identifier et sélectionner les éléments de régulation sont bien connus de l'homme du métier.

Pour la transformation des microorganismes comme les levures ou les bactéries, les éléments de régulation sont bien connus de l'homme du métier, et comprennent notamment des séquences promotrices, des activateurs de trancription, des peptides de transit, des séquences terminatrices et des codons start et stop.

Pour diriger l'expression et la sécrétion du peptide dans le milieu de culture de la levure, un fragment d'ADN codant l'héliomicine est intégré dans un vecteur navette qui comprend les éléments suivants :
- des marqueurs permettant de sélectionner les transformants. De préférence, on utilise le gène ura-3 pour la levure et le gène qui confère la résistance à l'ampicilline pour *E. coli.*
- une séquence nucléique permettant la réplication (origine de réplication) du plasmide dans la levure. De préférence on utilise l'origine de réplication du plasmide 2µ de levure,
- une séquence nucléique permettant la réplication (origine de réplication) du plasmide dans *E. coli,*
- une cassette d'expression constituée
   (1) d'une séquence de régulation promotrice. On peut utiliser toute séquence promotrice d'un gène s'exprimant naturellement dans la levure. De préférence, on utilise le promoteur du gène Mfα1 de *S. cerevisiae.*
   (2) d'une séquence codant pour un peptide signal (ou prépeptide) en association avec un peptide d'adressage (ou propeptide). Ces régions sont importantes pour la sécrétion correcte du peptide. De préférence, on utilise la séquence codant le pré-pro-peptide du précurseur du facteur Mfα1.
   (3) d'une séquence de régulation terminatrice ou de polyadénylation. De préférence, on utilise le terminateur de la phosphoglycérate kinase (PGK) de *S. cerevisiae.* Dans la cassette d'expression, la séquence codant l'héliomicine est insérée en aval de la séquence pré-pro et en amont du terminateur de la PGK.

Ces éléments ont été décrits dans plusieurs publications dont Reichhart et al., 1992, Invert. Reprod. Dev., 21, pp 15-24 et Michaut et al., 1996, FEBS Letters, 395, pp 6-10 .

De manière préférentielle, on transforme des levures de l'espèce *S*. *cerevisiae* avec le plasmide d'expression par la méthode à l'acétate de lithium (Ito et al., 1993, J. Bacteriol, 153, pp 163-168). Les levures transformées sont sélectionnées sur un milieu gélosé sélectif qui ne contient pas d'uracile. La production en masse des levures transformées est réalisée par culture pendant 24h à 48 h dans un milieu liquide sélectif.

La transformation de microorganismes permet de produire de l'héliomicine à plus large échelle. La présente invention concerne donc également un procédé de préparation de l'héliomicine, comprenant les étapes de culture d'un micro organisme transformé comprenant un gène codant pour l'héliomicine tel que défini ci-dessus dans un milieu de culture approprié, puis l'extraction et la purification totale ou partielle de l'héliomicine obtenue.

De manière préférée, lors de l'extraction de l'héliomicine produite par les levures, on élimine les levures par centrifugation et on met en contact le surnageant de culture avec une solution acide qui peut être une solution d'un acide minéral ou organique comme par exemple l'acide chlorhydrique ou de l'acide acétique. L'extrait obtenu est ensuite centrifugé à froid à une vitesse de 4000 à 10.000 rpm à 4°C, pendant 30 à 60 min.

La purification de l'héliomicine peut être précédée d'une étape de fractionnement du surnageant obtenu suite à l'étape d'extraction. De manière préférée, au cours de l'étape de fractionnement, l'extrait est déposé sur de la phase inverse pour réaliser une extraction en phase solide. Le lavage des molécules solubles dans l'eau est effectué avec une solution acide diluée et l'élution des molécules hydrophobes avec un éluant approprié. On obtient de bons résultats avec de l'acide trifluoroacétique pour le lavage et un éluant contenant des quantités croissantes d'acétonitrile en solution acide diluée.

De manière préférée la purification de l'héliomicine est effectuée en deux temps : une HPLC en échange de cations suivie d'une HPLC en phase inverse avec un éluant convenable qui peut être différent ou identique à celui de la phase précédente. Les différentes étapes de la purification sont suivies par un test d'inhibition de croissance fongique en milieu liquide. De préférence, le test est effectué avec le champignon *Neurospora crassa.*

La séquence de l'héliomicine produite par les levures transformées est analysée selon la méthode de séquençage par dégradation d'Edman et par spectrométrie de masse. La caractérisation structurale est réalisée directement sur le peptide produit, sur le peptide modifié par réduction/alkylation ainsi que sur des fragments du peptide. La séquence peptidique et la masse moléculaire de l'héliomicine produite ont été comparées avec celles de l'héliomicine native extraite de l'hémolymphe *d'H. virescens.* Les résultats montrent que les deux molécules présentent la même structure primaire. La détermination de la position des ponts disulfure indique que l'arrangement des ponts disulfures est identique dans les deux peptides, natif et produit par le microorganisme transformé.

L'invention concerne plus particulièrement la transformation des plantes. Comme séquence de régulation promotrice dans les plantes, on peut utiliser toute séquence promotrice d'un gène s'exprimant naturellement dans les plantes en particulier un promoteur d'origine bactérienne, virale ou végétale tel que, par exemple, celui d'un gène de la petite sous-unité de ribulose-biscarboxylase/oxygénase (RuBisCO) ou d'un gène de virus de plante tel que, par exemple, celui de la mosaïque du choux fleur (CAMV 19S ou 35S), ou un promoteur inductible par les pathogènes comme le PR-la du tabac, tout promoteur convenable connu pouvant être utilisé. De préférence on a recours à une séquence de régulation promotrice qui favorise la surexpression de la séquence codante de manière constitutive ou induite par l'attaque d'un pathogène, tel que par exemple, celle comprenant au moins un promoteur d'histone tel que décrit dans la demande EP 0 507 698.

Selon l'invention, on peut également utiliser, en association avec la séquence de régulation promotrice, d'autres séquences de régulation, qui sont situées entre le promoteur et la séquence codante, telles que des activateurs de transcription ("enhancer"), comme par exemple l'activateur de translation du virus de la mosaïque du tabac (TMV) décrit dans la demande WO 87/07644, ou du virus etch du tabac (TEV) décrit par Carrington & Freed.

Comme séquence de régulation terminatrice ou de polyadénylation, on peut utiliser toute séquence correspondante d'origine bactérienne, comme par exemple le terminateur nos *d'Agrobacterium tumefaciens,* ou encore d'origine végétale, comme par exemple un terminateur d'histone tel que décrit dans la demande EP 0 633 317.

Selon la présente invention, le gène chimère peut également être associé à un marqueur de sélection adapté à l'organisme hôte transformé. De tels marqueurs de sélection sont bien connus de l'homme du métier. Il pourra s'agir d'un gène de résistance aux antibiotiques, ou encore un gène de tolérance aux herbicides pour les plantes.

La présente invention concerne également un vecteur de clonage ou d'expression pour la transformation d'un organisme hôte contenant au moins un gène chimère tel que défini ci-dessus. Ce vecteur comprend outre le gène chimère ci-dessus, au moins une origine de réplication. Ce vecteur peut être constitué par un plasmide, un cosmide, un bactériophage ou un virus, transformés par l'introduction du gène chimère selon l'invention. De tels vecteurs de transformation en fonction de l'organisme hôte à transformer sont bien connus de l'homme du métier et largement décrits dans la littérature.

Pour la transformation des cellules végétales ou des plantes, il s'agira notamment d'un virus qui peut être employé pour la transformation des plantes développées et contenant en outre ses propres éléments de réplication et d'expression. De manière préférentielle, le vecteur de transformation des cellules végétales ou des plantes selon l'invention est un plasmide.

L'invention a encore pour objet un procédé de transformation des organismes hôtes, en particulier des cellules végétales par intégration d'au moins un fragment d'acide nucléique ou un gène chimère tels que définis ci-dessus, transformation qui peut être obtenue par tout moyen connu appropne. amplement décrit dans la littérature spécialisée et notamment les références citées dans in présente demande, plus particulièrement par le vecteur selon l'invention.

Une série de méthodes consiste a bombarder des cellules, des protoplastes ou des tissus avec des particules auxquelles sont accrochées les séquences d'ADN. Une autre série de méthodes consiste à utiliser comme moyen de transfert dans la plante un gène chimère inséré dans un plasmide Ti *d'Agrobacterium tumefaciens* ou Ri *d'Agrobacterium rhizogenes.*

D'autres méthodes peuvent être utilisées telles que la micro-injection ou l'électroporation, ou encore la précipitation directe au moyen de PEG.

L'homme du métier fera le choix de la méthode appropriée en fonction de la nature de l'organisme hôte, en particulier de la cellule végétale ou de la plante.

La présente invention a encore pour objet les organismes hôtes, en particulier cellules végétales ou plantes, transformés et contenant une quantité efficace d'un gène chimère comprenant une séquence codante pour l'héliomicine définie ci-dessus.

La présente invention a encore pour objet les plantes contenant des cellules transformées, en particulier les plantes régénérées à partir des cellules transformées. La régénération est obtenue par tout procédé approprié qui dépende de la nature de l'espèce, comme par exemple décrit dans les références ci-dessus.

Pour les procédés de transformation des cellules végétales et de régénération des plantes, on citera notamment les brevets et demandes de brevet suivants: US 4,459,355, US 4,536,475, US 5,464,763, US 5,177,010, US 5,187,073, EP 267,159, EP 604 662, EP 672 752, US 4,945,050, US 5,036,006, US 5,100,792, US 5,371,014, US 5,478,744, US 5,179,022, US 5,565,346, US 5,484,956, US 5,508,468, US 5,538,877, US 5,554,798, US 5,489,520, US 5,510,318, US 5,204,253, US 5,405,765, EP 442 174, EP 486 233, EP 486 234, EP 539 563, EP 674 725, WO 91/02071 et WO 95/06128.

La présente invention concerne également les plantes transformées issues de la culture et/ou du croisement des plantes régénérées ci-dessus, ainsi que les graines de plantes transformées, contenant les acides nucléiques et gènes chimères selon l'invention.

Les plantes ainsi transformées sont résistantes à certaines maladies, en particulier à certaines maladies fongiques ou bactériennes. De ce fait, la séquence d'ADN codant pour l'héliomicine peut être intégrée avec pour objectif principal la réalisation de plantes résistantes aux dites maladies, l'héliomicine étant efficace contre des maladies fongiques telles que celles causées par *Cercospora,* en particulier *Cercospora beticola, Cladosporium* en particulier *Cladosporium herbarum, Fusarium,* en particulier *Fusarium culmorum* ou *Fusarium graminearum,* ou par *Phytophthora,* en particulier *Phytophtora cinnamomi.*

Le gène chimère pourra comprendre également et de manière avantageuse au moins un marqueur de sélection, tel qu'un ou plusieurs gènes de tolérance aux herbicides.

La séquence d'ADN codant pour l'héliomicine peut également être intégrée comme marqueur de sélection lors de la transformation de plantes avec d'autres séquences codant pour d'autres peptides ou protéines d'intérêt, comme par exemple des gènes de tolérance aux herbicides.

De tels gènes de tolérance aux herbicides sont bien connus de l'homme du métier et notamment décrits dans les demandes de brevet EP 115 673, WO 87/04181, EP 337 899, WO 96/38567 ou WO 97/04103.

Bien entendu, les cellules et plantes transformées selon l'invention peuvent comprendre outre la séquence codant pour l'héliomicine, d'autres séquences hétérologues codant pour des protéines d'intérêt comme d'autres peptides complémentaires susceptibles de conférer à la plante des résistances à d'autres maladies d'origine bactérienne ou fongique, et/ou d'autres séquences codant pour des protéines de tolérance aux herbicides et/ou d'autres séquences codant pour des protéines de résistance aux insectes, comme les protéines *Bt* notamment.

Les autres séquences peuvent être intégrées au moyen du même vecteur comprenant un gène chimère, lequel comprend une première séquence codant pour l'héliomicine et au moins une autre séquence codant pour un autre peptide ou protéine d'intérêt.

Elles peuvent également être intégrées au moyen d'un autre vecteur comprenant au moins la dite autre séquence, selon les techniques usuelles définies ci-dessus.

Les plantes selon l'invention peuvent encore être obtenues par croisement de parents, l'un portant le gène selon l'invention codant pour l'héliomicine, l'autre portant un gène codant pour au moins un autre peptide ou protéine d'intérêt.

Parmi les séquences codant pour d'autres peptides antifongiques, on peut citer celle codant pour la drosomycine, décrite dans la demande de brevet FR 2 725 992 et par Fehlbaum & coll. (1994), et dans la demande de brevet non publiée FR 97 09115 déposée le 24 juillet 1997, ou celle codant pour l'androctonine décrite dans la demande de brevet FR 2 745 004 et dans la demande de brevet non publiée FR 97 10362 déposée le 20 août 1997.

La présente invention concerne enfin un procédé de culture des plantes transformées selon l'invention, le procédé consistant à planter les graines des dites plantes transformées dans une surface d'un champ approprié pour la culture des dites plantes, à appliquer sur la dite surface du dit champ une composition agrochimique, sans affecter de manière substantielle les dites graines ou les dites plantes transformées, puis à récolter les plantes cultivées lorsqu'elles arrivent à la maturité souhaitée et éventuellement à séparer les graines des plantes récoltées.

Par composition agrochimique, on entend selon l'invention toute composition agrochimique comprenant au moins un produit actif ayant l'une des activités suivantes, herbicide, fongicide, bactéricide, virucide ou insecticide.

Selon un mode préférentiel de réalisation du procédé de culture selon l'invention, la composition agrochimique comprend au moins un produit actif ayant au moins une activité fongicide et/ou bactéricide, plus préférentiellement présentant une activité complémentaire de celle de l'héliomicine produite par les plantes transformées selon l'invention.

Par produit présentant une activité complémentaire de celle de l'héliomicine, on entend selon l'invention un produit présentant un spectre d'activité complémentaire, c'est à dire un produit qui sera actif contre des attaques de contaminants (champignons, bactéries ou virus) insensibles à l'héliomicine, ou encore un produit dont le spectre d'activité recouvre celui de l'héliomicine, totalement ou en partie, et dont la dose d'application sera diminuée de manière substantielle du fait de la présence de l'héliomicine produite par la plante transformée.

Les exemples ci-après permettent d'illustrer la présente invention, sans toutefois en limiter la portée.

### Exemple I : Isolement et caractérisation de l'héliomicine à partir de l'hémolymphe prélevée chez des larves immunisées du lépidoptère H. virescens

### Exemple I.1 : Isolement

### 1-1 Induction de la synthèse biologique d'une substance antifongique dans l'hémolymphe d' H. virescens

Les larves matures de 5ème stade du lépidoptère *H. virescens* ont été immunisées à l'aide d'une aiguille (30 ga) préalablement plongée dans un culot de bactéries à Gram positif (*M. luteus*) et à Gram négatif (*E*. *coli* 1106) préparé à partir de cultures réalisées en milieu de Luria-Bertani durant 12 heures à 37°C. Les animaux ainsi infectés ont été conservés individuellement dans des tubes contenant un milieu nutritif à base d'agar pendant 24 heures entre 20°C et 23°C. Avant le prélèvement de l'hémolymphe les larves ont été refroidies sur de la glace.

### 1-2 Préparation du plasma

L'hémolymphe (environ 30 µl par larve) a été collectée par excision d'un appendice abdominal et recueillie dans des tubes de microcentrifugation en polypropylène de 1,5 ml refroidis dans de la glace et contenant de l'aprotinine comme inhibiteur de protéases (20µg/ml en concentration finale) et de la phénylthiourée comme inhibiteur de la mélanisation (concentration finale de 20µM). L'hémolymphe (2 ml) ainsi collectée à partir des larves immunisées a été centrifugée à 14000 g pendant 1 min à 4 °C afin de retirer les hémocytes. L'hémolymphe dépourvue des cellules sanguines a été conservée à -20°C jusqu'à son utilisation.

### 1-3 Acidification du plasma

Après décongélation rapide, le plasma d' *H. virescens* a été acidifié jusqu'à pH 3 avec une solution d'acide trifluoroacétique à 1%. L'extraction en condition acide du peptide a été réalisée pendant 30 min sous agitation légère dans un bain d'eau glacée. L'extrait obtenu a été ensuite centrifugé à 4°C pendant 30 min à 10 000g.

### I-4 Purification des peptides

### a) Prépurification par extraction en phase solide

Une quantité d'extrait équivalente à 2ml d'hémolymphe a été déposée sur un support de phase inverse, tel que commercialisé sous la forme de cartouche (Sep-Pak™ C18, Waters Associates), équilibré avec de-l'eau acidifiée (TFA 0,05 %). Les molécules hydrophiles ont été éliminées par un simple lavage avec de l'eau acidifiée. L'élution du peptide a été réalisée par une solution d'acétonitrile à 40% préparée dans le TFA 0,05%. La fraction éluée à 40% d'acétonitrile a été séchée sous vide dans le but d'éliminer l'acétonitrile et le TFA puis elle a été reconstituée dans de l'eau ultrapure stérile avant d'être soumise à la première étape de purification.

### b) Purification par chromatographie liquide à haute performance (HPLC) sur colonne de phase inverse.

- **première étape :** la fraction contenant le peptide a été analysée par chromatographie de phase inverse sur une colonne semi-préparative Aquapore RP-300 C₈ (Brownlee™, 220 x 70 mm, 300 Å), l'élution a été réalisée par un gradient linéaire d'acétonitrile de 2 à 60% dans le TFA 0.05% pendant 120 minutes à un débit constant de 1.5ml/min. Les fractions ont été collectées manuellement en suivant la variation de l'absorbance à 225 nm et 254 nm. Les fractions recueillies ont été asséchées sous vide, reconstituées avec de l'eau ultrapure et analysées pour leur activité antifongique en utilisant le test décrit ci-dessous.
- **deuxième étape :** la fraction antifongique correspondant au peptide a été analysée sur une colonne analytique de phase inverse Aquapore RP-300 C₈ (Brownlee™, 220 x 4,6 mm, 300 Å), en utilisant un gradient linéaire diphasique d'acétonitrile de 2% à 22% en 10 min et de 22 à 32% en 50 min dans le TFA 0,05% avec un débit constant de 0,8 ml/min. Les fractions ont été collectées manuellement en suivant la variation de l'absorbance à 225 nm et 254 nm. Les fractions recueillies ont été asséchées sous vide, reconstituées avec de l'eau ultrapure et analysées pour leur activité antifongique dans les conditions décrites ci-dessous.
- **troisième étape :** la fraction antifongique contenant le peptide a été purifiée jusqu'à homogénéité sur une colonne de phase inverse Narrowbore Delta-Pak™ HPIC₁₈ (Waters Associates, 150 x 2,2 mm) eu utilisant un gradient linéaire diphasique d'acétonitrile de 2% à 24% en 10 min et de 24 à 44% en 100 min dans le TFA 0,05% avec un débit constant de 0,25 ml/ min à une température contrôlée de 30°C. Les fractions ont été collectées manuellement en suivant la variation de l'absorbance à 225 nm. Les fractions recueillies ont été asséchées sous vide, reconstituées avec de l'eau ultrapure filtrée et analysées pour leur activité antifongique.

### Exemple I.2 : caractérisation structurale du peptide

### 2-1 Vérification de la pureté par électrophorèse capillaire de zone

La pureté du peptide antifongique a été vérifiée par électrophorèse capillaire de zone sur un modèle 270-HT (*PE*Applied Biosystems division de Perkin Elmer). 1nl d'une solution à 50µM de peptide purifié a été injecté sous assistance par le vide dans un capillaire de silice (72 cm x 50 µm) et l'analyse a été réalisée en tampon citrate 20 mM à pH 2,5. L'électrophorèse a été réalisée a 20 kV de l'anode à la cathode pendant 20 min à 30°C. La migration a été enregistrée à 200 nm.

### 2-2 Détermination du nombre des cystéines : réduction et S-pyridyléthylation.

Le nombre de résidus cystéine a eté déterminé sur le peptide natif par réduction et S-pyridyléthylation. 100 pmoles de peptide natif ont été réduites dans 40 µl de tampon Tris/HCl 0,5 M, pH 7,5 contenant 2 mM JFDTA et 6 M de chlorure de guanidinium en présence de 2 µl de dithiothréitol 2.2 M le milieu réactionnel a été placé sous atmosphère d'azote. Après 60 min d'incubation a l'obscurité, 2 µl de 4-vinylpyridine fraîchement distillée ont été ajoutés à la réaction qui a été alors incubée durant 10 min à 45°C à l'obscurité et sous atmosphère d'azote. Le peptide pyridyléthylé a été ensuite séparé des constituants du milieu réactionnel par chromatographie de phase inverse en utilisant un gradient linéaire d'acétonitrile en présence de TFA 0,05%.

### 2-3 Détermination de la masse du peptide natif, du peptide S-pyridyléthylé et des fragments de protéolyse .par spectrométrie de masse MALDI-TOF (Matrix Assisted Laser Desorption Ionization-Time Of Flight).

Les mesures de masses ont été effectuées sur un spectromètre de masse MALDI-TOF Bruker Biflex (Bremen, Allemagne) en mode linéaire positif. Les spectres de masse ont été calibrés de façon externe avec un mélange standard de peptides de m/z connues, respectivement 2199.5 Da, 3046.4 Da et 4890.5 Da. Les différents produits à analyser ont été déposés sur une fine couche de cristaux d'acide α-eyano-4-hydroxycinnamique obtenue par une évaporation rapide d'une solution saturée dans l'acétone. Après séchage sous un léger vide, les échantillons ont été lavés par une goutte d'acide trifluoroacétique à 0,1% avant d'être introduits dans le spectromètre de masse.

### 2-4 Séquençage par dégradation d'Edman.

Le séquençage automatique par dégradation d'Edman du peptide natif, du peptide S-pyridyléthylé et des différents fragments obtenus après les différents clivages protéolytiques et la détection des dérivés phénylthiohydantoines ont été réalisés sur un séquenceur ABI473A (*PE*Applied Biosystems division de Perkin Helmer).

### 2-5 Clivages protéolytiques.

### - Confirmation de la séquence peptidique dans la région C-terminale.

200 pmoles de peptide réduit et S-pyridyléthylé ont été incubées en présence de 5 pmoles d'endoprotéinase-Lys-C (*Acromobacter* protease 1, clivage spécifique des résidus lysine du côté C-terminal, Takara. Otsu) selon les conditions préconisées par le fournisseur (Tris HCl 10 mM pH 9 en présence de Tween 20 à 0,01%). Après arrêt de la réaction avec du TFA 1%, les fragments peptidiques ont été séparés par HPLC en phase inverse sur une colonne de type Narrowbore Delta-Pak™ HPIC₁₈ (Waters Associates, 150 x 2 mm) dans un gradient linéaire d'acétonitrile de 2 à 60% en 80 min dans le TFA 0,05% avec un débit de 0,2 ml/min et une température constante de 37°C. Les fragments obtenus ont été analysés par spectrométrie de masse MALDI-TOF et le peptide correspondant au fragment C-terminal a été séquencé par dégradation d'Edman.

### -Détermination de l'arrangement des ponts disulfure par protéolyse à la thermolysine.

Le peptide natif (8 µg) a été incubé durant 1 heure en présence de 4 µg de thermolysine (Boehringer Manheim, rapport thermolysine/peptide, 1/2 en poids : poids) à 37°C dans le tampon MES (N-éthylmorpholine) 0,1 M à pH 7 en présence de 2 mM de CaCl2. La réaction a été arrêtée par addition d'acide formique et les produits de la réaction ont été immédiatement séparés par chromatographie de phase inverse sur une colonne Narrowbore Delta-Pak™ HPIC₁₈ (Waters Associates, 150 x 2,2 mm) dans un gradient linéaire d'acétonitrile de 2 à 50% en 100 min dans du TFA 0,05% au débit de 0,2 ml/min à 30°C précédée d'une étape isocratique à 2 % d'acétonitrile pendant 10 min. Les fragments obtenues ont été analysés par spectrométrie de masse MALDI-TOF et séquencés par dégradation d'Edman.

### Exemple II : Expression de l'héliomicine dans la levure Saccharomyces cerevisiae.

Toutes les techniques employées ci-après sont des techniques standards de laboratoire. Les protocoles détaillés de ces techniques ont été notamment décrits dans Ausubel *et al.*

### Exemple II-1 Assemblage du gène synthétique

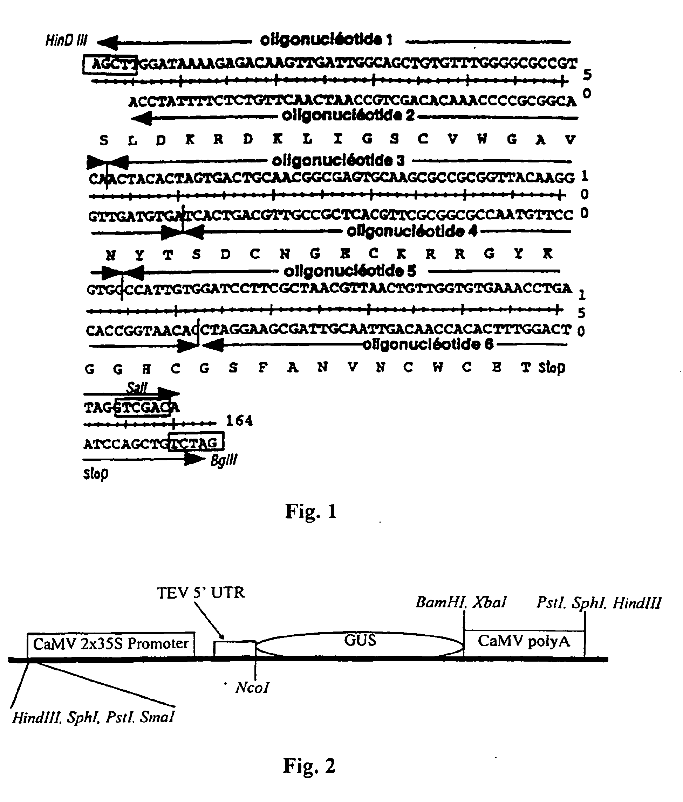

L'assemblage a été réalisé à partir de 6 oligonucléotides synthétiques codant pour les 44 acides aminés de l'héliomicine précédés des 5 acides aminés C-terminaux de la préproséquence du facteur a (Mfa1) de la levure. Les oligonucléotides représentés sur la figure 1 ont été choisis en tenant compte des codons préférentiels utilisés par *S. cerevisiae.*

L'assemblage s'est déroulé en plusieurs étapes :
- les oligonucléotides 2 à 5 ont été phosphorylés à leurs extrémités 5' par action de la polynucléotide kinase (New England Biolabs) ;
- les oligonucléotides 1 à 6 ont été mélangés, chauffés à 100°C et hybridés par diminution lente de la température à 25 °C pendant 3 heures ;
- les oligonucléotides hybridés ont été soumis à un traitement par la ligase du bactériophage T4 (New England Biolabs) pendant 15 heures à 15° C ;
- le bloc d'ADN résultant de l'hybridation des oligonucléotides représenté sur la figure 1, borné par les sites de restriction HinDIII et BglII, a été inséré dans le plasmide pBluescript SK+ (Stratagène) digéré par les enzymes HinDIII et BamHI (BgIII et BamHI sont compatibles). Le mélange de ligation a ensuite été utilisé pour transformer des cellules compétentes d' *E. coli* DH5α. (Stratagène). Plusieurs clones ont été analysés et séquencés. Un de ces clones qui présentait la séquence recherché a été appelé pSEA1.

### Exemple II-2 : Construction du vecteur pSEA2 qui permet la sécrétion de l'héliomicine synthétisée.

Le fragment d'ADN HinDIII-SaII du vecteur pSEA1, portant la séquence codant l'héliomicine ainsi que le fragment SphI-HinDIII du vecteur M13JM132 (Michaut et al., 1985, FEBS Letters, 395, pp 6-10) ont été insérés entre les sites SphI et SalI du plasmide pTG4812 (Michaut et al., 1996, FEBS Letters, 395, pp 6-10). Le fragment SphI-HinDIII du vecteur M13JM132 contient la séquence du promoteur du gène MFα1 de la levure ainsi que la séquence codant la région pré-pro du facteur MFα1. Dans le plasmide résultant, pSEA2, le gène synthétique de l'héliomicine se retrouve donc inséré entre les séquences pré-pro du facteur Mfα1 et le terminateur de transcription; cette construction doit donc assurer la maturation et la sécrétion de l'héliomicine.

### Exemple II-3 : Transformation d'une souche de S. cerevisiae par l'ADN du plasmide pSEA2 et analyse des transformants.

La souche de levure TGY 48.1 (MATa, ura3-D5,his,pral,prbl, prc1, cps1 ; Reichhart et al., 1992, Invert. Reprod. Dev. 21, pp 15-24) a été transformée par le plasmide pSEA2. Les transformants ont été sélectionnés à 29°C sur un milieu sélectif YNBG (0,67% yeast nitrogen base, 2% glucose), supplémenté avec 0,5 % de casamino acides et ne contenant pas d'uracile. Après transformation, plusieurs clones de levures, sélectionnés pour le caractère ura+, ont été mis en culture pendant 48 h à 29°C dans 50 ml de milieu sélectif. Après centrifugation (4000 g, 30 min, 4°C), le surnageant a été acidifié jusqu'à pH 3,5 avec de l'acide acétique, avant d'être déposé sur une cartouche Sep-Pak™ C₁₈, (Waters Associates) équilibrée avec de l'eau acidifiée (TFA 0,05 %). Les différentes protéines fixées sur la cartouche ont été éluées par des solutions de TFA 0,05% contenant des pourcentages croissants d'acétonitrile.

La fraction 40 %, présentant une activité antifongique, a été analysée en HPLC sur une colonne analytique de phase inverse Aquapore RP-300 C₈ (Brownlee™. 220 x 4.6 mm, 300 Å), en utilisant un gradient linéaire d'acétonitrile de 2% à 40% en 80 min dans le TFA 0,05% avec un débit constant de 0,8 ml/min. Les fractions ont été collectées manuellement en suivant la variation de l'absorbance à 225 nm et 254 nm. Les fractions recueillies ont été asséchées sous vide, reconstituées avec de l'eau ultrapure et analysées pour leur activité antifongique dans les conditions décrites dans l'exemple III. La caractérisation structurale du peptide a été réalisée comme décrit dans l'exemple 1.2.

### Exemple II-4 : Production d'héliomicine recombinante à l'échelle semi-préparative.

Un des clones de levure transformée exprimant l'héliomicine a été cultivé à 29°C pendant 24 h dans 100 ml de milieu sélectif. Cette préculture a ensuite été utilisée pour inoculer 4 1 de milieu sélectif et la culture a été réalisée pendant 48 h à 29 °C. Les levures ont été éliminées par centrifugation (4000 g, 30 min, 4°C). Le surnageant a été acidifié jusqu'à pH 3,5 avec de l'acide acétique, soumis à une deuxième centrifugation (4000 g, 30 min, 4°C) avant d'être déposé sur une colonne ouverte de phase inverse preparative C₁₈ (Waters Associates), 125 Å, 6 g de phase pour 500 ml de surnageant) équilibrée avec de l'eau acidifiée (TFA 0,05 %). Les molécules hydrophiles ont été éliminées par un lavage avec de l'eau acidifiée suivie d'un lavage avec une solution d'acétonitrile à 15% préparée dans le TFA 0,05%. L'élution du peptide a été réalisée par une solution d'acétonitrile à 40% préparée dans le TFA 0,05%. La fraction éluée à 40% d'acétonitrile a été lyophilisée puis reconstituée dans de l'eau ultrapure stérile avant d'être soumise à la première étape de purification.
- première étape de purification par HPLC : la fraction prépurifiée contenant l'héliomicine a été reconstituée dans une solution d'acétate d'ammonium à 25 mM, pH 3,4. Cet échantillon a été injecté sur une colonne préparative d'échange de cations Aquapore Cation Exchange (Brownlee™, 250 x 10 mm), en utilisant un gradient linéaire de NaCl de 0% à 100% en 90 min dans l'acétate d'ammonium 25 mM, pH 3,4 avec un débit constant de 2 ml/min. Les fractions recueillies ont été asséchées sous vide, reconstituées avec de l'eau ultrapure et analysées pour leur activité antifongique dans les conditions décrites ci-dessous.
- deuxième étape de purification par HPLC : l'héliomicine a été purifiée jusqu'à homogénéité par chromatographie sur une colonne de phase inverse semi-préparative Aquapore RP-300 C8 (Brownlee™, 220 x 7 mm, 300 Å), eu utilisant un gradient linéaire d'acétonitrile de 2% à 40% en 80 min dans le TFA 0,05% avec un débit constant de 2 ml/min.

### Exemple III : Test d'activité in vitro : mesure de l'activité antifongique par microspectrophotométric.

Cette méthodologie a été utilisée pour la mise en évidence des molécules antifongiques au cours des différentes étapes de purification, pour la détermination du spectre d'activité du peptide et pour la détermination de la concentration minimale inhibitrice (CMI) à laquelle le peptide a été actif. La CMI a été exprimée comme un intervalle de concentration [a] - [b] où [a] a été la concentration minimale où l'on observe un début de croissance et [b] la concentration pour laquelle aucune croissance n'a été observée. Des exemples de l'activité spécifique de l'héliomicine, vis-à-vis des champignons filamenteux et des levures, sont donnés dans les tableaux 1 et 2.

### Exemple III-1 : Test de détection d'activité contre les champignons filamenteux

L'activité antifongique a été détectée par un test d'inhibition de croissance en milieu liquide. Les spores des champignons à tester ont été mises en suspension dans un milieu de culture de type « Pomme de terre-Glucose ». De préférence, on utilise 12 g de milieu Potato Dextrose Broth (Difco) pour 1 1 d'eau déminéralisée. Deux antibiotiques ont été rajoutés au milieu de culture : la tétracycline (concentration finale de 10 µg/ml) et la céfotaxime (100µg/ml). On dépose 10 µl de chaque fraction à analyser dans des plaques de microtitration en présence de 90 µl de milieu de culture contenant les spores (à une concentration finale de 104 spores/ml). L'incubation a été réalisée en chambre humide à 30°C durant 48 heures. La croissance fongique a été observée au microscope photonique après 24 h et quantifiée après 48 heures par mesure de l'absorbance à 600 nm à l'aide d'un spectrophotomètre lecteur de plaque de microtitration..
- champignons filamenteux testes : *Aspergillus fumigatus* (don du Dr H. Koenig, Hôpital civil, Strasbourg) ; *Nectria haematococca, Fusarium culmorum, Trichoderma viride* (mycothèque de l'Université Catholique de Leuven, Belgique); *Neurospora crassa, Fusarium oxysporum,* (mycothèque de la Société Clause, Paris).

Les résultats du test d'activité de l'héliomicine contre les champignons filamenteux sont reportés dans le Tableau 1 ci-dessous.

**Tableau 1 : activité de l'héliomicine contre les champignons filamenteux**

| **Champignons** | **CMI de l'héliomicine (µM)** |
|---|---|
| Neurospora crassa | 0,1-0,2 |
| Fusarium culmorum | 0,2-0,4 |
| Fusarium oxysporum | 1,5-3 |
| Nectria haematococca | 0,4-0,8 |
| Trichoderma viride | 1,5-3 |
| Aspergillus fumigatus | 6-12,5 |

### Exemple III-2 : Test de détection d'activité contre les levures

Les différentes souches de levure ont été mises en incubation dans un milieu de culture de type « Sabouraud » et incubée à 30°C pendant 24 h sous agitation lente. L'échantillon à tester (10 µl) a été déposé dans des puits de plaque de microtitration dans lesquels ont été ajoutés 90 µl d'une culture diluée de levure dont la densité a été ajustée à DO 600 = 0,001. On évalue la croissance par la mesure de l'absorbance à 600 nm à l'aide d'un spectrophotomètre lecteur de plaque de microtitration.
- levures testées : *Candida albicans, C. glabrata, C. tropicalis, C. krusei, C. inconspicua, Cryptococcus neoformans, Cryp. albidus, Saccharomyces cerevisiae* (don du Dr H. Koenig, Hôpital civil, Strasbourg)

Les résultats du test d'activité de l'héliomicine contre les levures sont reportés dans le Tableau 2 ci-dessous.

**Tableau 2 : activité de l'héliomicine contre les levures.**

| **Levures** | **CMI de l'héliomicine (µM)** |
|---|---|
| Candida albicans | 2,5-5 |
| Candida tropicalis | 2,5-5 |
| Candida krusei | 10-20 |
| Candida inconspicua | 5-10 |
| Cryptococcus neoformans | 2,5-5 |
| Cryptococcus albidus | 5-10 |

Ces résultats montrent l'excellente activité antifongique du peptide selon l'invention.

### Exemple IV: Préparation d'une plante transformée comprenant un gène codant pour l'héliomicine

Cet exemple décrit la préparation de la séquence codant pour l'héliomicine pour son expression dans une cellule végétale, du gène chimère, du vecteur d'intégration et des plantes transformées. Les figures 2 à 6 en annexe décrivent les structures schématiques de certains plasmides préparés pour la construction des gènes chimères. Dans ces figures, les différents sites de restriction sont marqués en *italiques.*

Toutes les techniques employées ci-après sont des techniques standard de laboratoire. Les protocoles détaillés de ces techniques sont notamment décrits dans Ausubel & coll.

### Exemple IV-1 : Construction des gènes chimères pour la transformation de plantes pRPA-MD-P: Création d'un plasmide contenant le signal peptide du gène PR-1α du tabac.

Les deux oligonucléotides synthétiques complémentaires Oligo 7 et Oligo 8 ci-après, sont hybridés à 65 °C pendant 5 minutes puis par diminution lente de la température à 30°C pendant 30'.

Après hybridation entre l'Oligo 7 et l'Oligo 8, l'ADN resté simple brin sert de matrice au fragment klenow de la polymérase 1 de *E. coli* (dans les conditions standard préconisées par le fabriquant (New England Biolabs)) pour la création de l'oligonucléotide double brin à partir de l'extrémité 3' de chaque oligo. L'oligonucléotide double brin obtenu est ensuite digéré par les enzymes de restriction *SacII* et *NaeI* et cloné dans le plasmide pBS II SK(-) (Stratagene) digéré par les même enzymes de restriction. On obtient alors un clone comprenant la région codant pour peptide signal du gène PR-1α du tabac (SEQ ID NO 4).

### pRPA-PS-PR1a-hélio: Création d'une séquence codant pour l'héliomicine fusionée au signal peptide PR-1α sans région non transcrite en 3'.

Les deux oligonucléotides synthétiques complémentaires de séquences Oligo 9 et Oligo 10 selon les conditions opératoires décrites pour pRPA-MD-P.

Après hybridation entre l'Oligo 9 et l'Oligo 10, l'ADN resté simple brin sert de matrice au fragment klenow de la polymérase 1 de *E. coli* (dans les conditions standard préconisées par le fabriquant (New England Biolabs)) pour la création de l'oligonucléotide double brin à partir de l'extrémité 3' de chaque oligo. Cet oligonucléotide double brin contenant la partie codante de l'héliomicine (SEQ ID NO 2) est ensuite cloné directement dans le plasmide pRPA-MD-P qui a été digéré avec l'enzyme de restriction *NaeI.* L'orientation correcte du clone obtenu est vérifiée par séquençage. On obtient alors un clone comprenant la région codant pour la protéine de fusion PR-la-héliomicine située entre les sites de restriction *NcoI* à l'extrémité N-terminale et *ScaI, SacII* et *BamHI* à l'extrémité C-terminale (SEQ ID NO 3).

### pRPA-RD-239: Création d'un vecteur d'expression dans les plantes comprenant la séquence codant pour la protéine de fusion PR-1a-héliomicine.

Le plasmide pRTL-2 GUS, dérivé du plasmide pUC-19, a été obtenu auprès du Dr. Jim Carrington (Texas A&M University, non décrit). Ce plasmide dont la structure schématique est représentée sur la figure 2, contient le promoteur CαMV 35S dupliqué isolé du virus de la mosaïque du choux fleur (Promoteur CaMV 2x35S; Odell & coll., 1985) qui dirige l'expression d'un ARN contenant séquence non traduite en 5' du virus etch du tabac (TEV 5' UTR; Carrington & Freed, 1990), le gène de la β-glucuronidase d' *E. coli* (GUS Jefferson & coll., 1987) suivi du site de polyadenylation de l'ARN 35S de CaMV (CaMV polyA; Odell & coll., 1985).

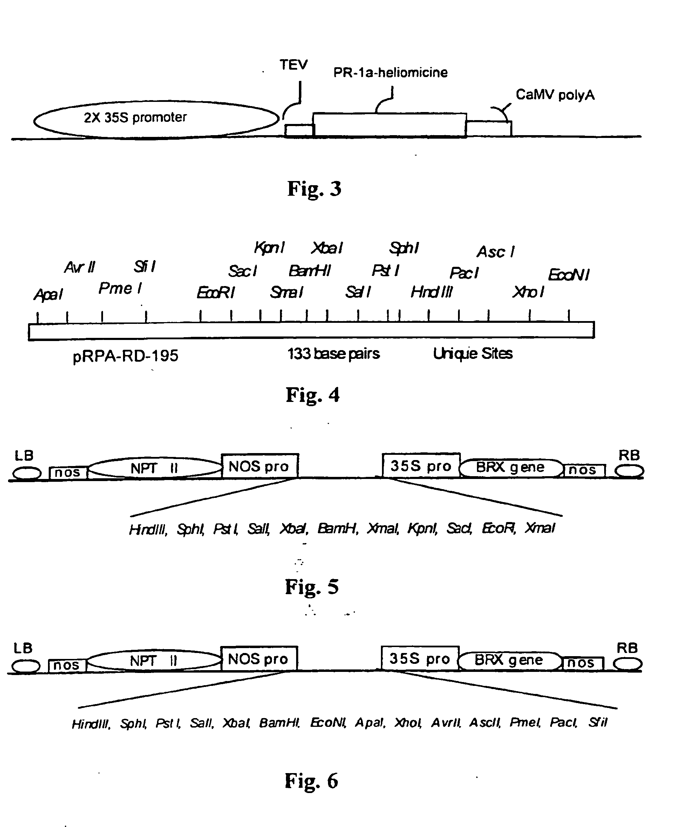

Le plasmide pRTL-2 GUS est digéré avec les enzymes de restriction *NcoI* et *BamHI* et le grand fragment d'ADN est purifié. Le plasmide pRPA-PS-PR1α-hélio est digéré avec les enzymes de restriction *NcoI* et *BamHI* et le petit fragment d'ADN contenant la région codant pour la protéine de fusion PR-1α-héliomicine est purifié. Les deux fragments d'ADN purifiés sont ensuite liés ensemble dans une cassette d'expression dans les plantes qui synthétise une protéine de fusion PR-1α-héliomicine. La structure schématique de cette cassette d'expression est représentée sur la figure 3. « PR-1α-héliomicine » représente la région codante pour la protéine de fusion PR-1α-héliomicine de pRPA-RD-239. L'héliomicine est transportée vers la matrice extra-cellulaire de la plante par l'action du peptide signal PR-1α.

### pRPA-RD-195: Création d'un plasmide contenant un site de clonage multiple modifié.

Le plasmide pRPA-RD-195 est un plasmide dérivé du pUC-19 qui contient un site de clonage multiple modifié. Les oligonucléotides synthétiques complémentaires Oligo 11 et Oligo 12 ci-après, sont hybridés et rendus double brin selon la procédure décrite pour pRPA-MD-P.

L'oligonucléotide double brin obtenu est ensuite lié dans pUC-19 qui a été préalablement digéré avec les enzymes de restriction *EcoRI* et *HindIII* et rendu bouts francs en employant le fragment klenow de l'ADN polymérase 1 de *E*. *coli.* On obtient un vecteur contenant de multiples sites de clonage pour faciliter l'introduction des cassettes d'expression dans un plasmide vecteur *d'Agrobacterium tumefaciens.* La structure schématique de ce site de clonage multiple est représentée sur la figure 4.

### nRPA-RD-240: Introduction de la cassette d'expression de PR-la-héliomicine de pRPA-RD-239 dans pRPA-RD-195.

Le plasmide pRPA-RD-239 est digéré avec l'enzyme de restriction *PstII.* Le fragment d'ADN contenant la cassette d'expression de PR-la-héliomicine est purifié. Le fragment purifié est ensuite lié dans pRPA-RP-195 qui a été préalablement digéré avec l'enzyme de restriction *PstII* et déphosphorylé avec la phosphatase intestinale de veau.

### pRPA-RD-174: Plasmide dérivé de pRPA-BL-150A (EP 0 508 909) contenant le gène de tolérance au bromoxynil de pRPA-BL-237 (EP 0 508 909).

Le gène de tolérence au bromoxynil est isolé de pRPA-BL-237 par une amplification génique par PCR. Le fragment obtenu est à bouts francs et est cloné dans le site *EcoRI* de pRPA-BL-150A qui a été rendu bouts francs par l'action de la polymérase klenow dans des conditions standard. On obtient un vecteur d'*Agrobacterium tumefaciens* qui contient le gène de tolérance au bromoxynil à proximité de sa bordure droite, un gène de tolérance à la kanamycine à proximité de sa bordure gauche et un site de clonage multiple entre ces deux gènes.

La structure schématique de pRPA-RD-174 est représentée sur la figure 5. Sur cette figure, "nos" représente le site de polyadenylation de la nopaline synthase *d'Agrobacterium tumefaciens* (Bevan & coll., 1983), "NOS pro" représente le promoteur de la nopaline synthase *d'Agrobacterium tumefaciens* (Bevan & coll., 1983), "NPT II" représente le gène de la néomycine phosphotransphérase du transposon Tn5 de *E. coli* (Rothstein & coll., 1981), "35S pro" représente le promoteur 35S isolé du virus de la mosaïque du choux fleur (Odell & coll., 1985), "BRX" représente le gène de la nitrilase isolé de *K. ozaenae* (Stalker & coll., 1988), "RB" et "LB" représentent respectivement les bordures droite et gauche de la séquence d'un plasmide Ti d'*Agrobacterium tumefaciens.*

### pRPA-RD-184: Addition d'un nouveau site de restriction, unique, dans pRPA-RD-174.

Les oligonucléotides synthétiques complémentaires Oligo 13 et Oligo 14 ci-après, sont hybridés et rendus double brin selon la procédure décrite pour pRPA-MD-P.

L'oligonucléotide double brin hybridé (95 paires de bases) est purifié après séparation sur un gel d'agarose (3 % Nusieve, FMC). Le plasmide pRPA-RD-174 est digéré avec l'enzyme de restriction *XmaI* et le grand fragment d'ADN est purifié. Les deux fragments d'ADN obtenus sont ensuite liés

On obtient un plasmide dérive de pRPA-RD-174 comprenant d'autres sites de restriction entre le gène de tolérance au bromoxynil et le gène de la kanamycine marqueur de sélection.

La structure schématique du plasmide pRPA-RD-184 est représentée sur la figure 6 où les termes "nos". "NPT II". "NOS pro", "35S pro", "BRX gene", "RB" et "LB" ont la même signification que pour la figure 5.

### pRPA-RD-241: Création d'un vecteur d'Agrobacterium tumefaciens contenant la construction du gène codant pour l'héliomicine dirigée vers la matrice extracellulaire.

La plasmide pRPA-RD-240 est digéré avec les enzymes de restriction *SfiII* et *AscI* et le fragment d'ADN contenant le gène de PR-la-héliomicine est purifié. Le plasmide pRPA-RD-184 est digéré avec les même enzymes de restriction. Le fragment d'ADN contenant la cassette d'expression de PR-la-héliomicine est ensuite liée dans pRPA-RD-184. On obtient ainsi un vecteur d'*Agrobacterium tumefaciens* contenant la séquence codant pour la protéine de fusion PR-1α-héliomicine qui conduit à l'expression de l'héliomicine dans la matrice extracellulaire de la plante.

### Exemple IV - 2 Création d'une cassette d'expression promoteur CsVMV - peptide signal PG1 - héliomicine - terminateur Nos

### pRPA - NP4 : Création d'un plasmide contenant le peptide signal du gène PG1 de polygalacturonase de maïs (Genbank, accession n° X57627).

Les deux oligonucléotides synthétiques partiellement complémentaires Oligo 13 et Oligo 14 ci-après sont hybridés à 65°C pendant 5 minutes puis par diminution lente de la température à 30°C pendant 30 minutes.

Après hybridation entre l'Oligo 13 et l'Oligo 14, l'ADN resté simple brin sert de matrice au fragment Klenow de la polymérase 1 de E. coli (dans les conditions standard préconisées par le fabricant (New England Biolabs) pour la création de l'oligonucléotide double brin à partir de l'extrêmité 3' de chaque oligo. L'oligonucléotide double brin obtenu est ensuite digéré par les enzymes de restriction XbaI et EcoRI, puis cloné dans le plasmide pBS II SK(-) (Stratagene) digéré par les mêmes enzymes de restriction. On obtient alors un clone contenant la région codant pour les 22 acides aminés du peptide signal du gène PG1. et qui peut être fusionné au cadre de lecture d'autres protéines au niveau du site SfoI (SEQ ID NO 15).

### pRPA - NP5 : Création d'une séquence codant pour l'héliomicine fusionnée au peptide signal du gène PG1

La région codant pour l'héliomicine a été amplifiée par PCR à partir du clone pRPA - PS - PRla - hélio (SEQ ID NO 3) avec l'enzyme thermostable Pfu (Stratagene) selon les conditions standard préconisées par le fabriquant. Les oligonucléotides synthétiques utilisés pour cette amplification sont :

Le produit de PCR a été digéré par l'enzyme de restriction XhoI et cloné dans le vecteur pRPA - NP4 digéré par les enzymes de restriction SfoI et XhoI. Le clone résultant comprend donc la région codant pour le peptide signal du gène PG1 fusionnée dans le même cadre de lecture avec la séquence codant pour l'héliomicine (SEQ ID NO 18).

### pRPA - NP6 : Création d'une cassette d'expression de l'héliomicine dans un vecteur de transformation

Le vecteur d'expression et de transformation pILTAB 357 est dérivé du vecteur binaire pBin19. Il contient le promoteur CsVMV (Verdaguer et al. 1996, Plant Mol. Biol. 31, 1129-1139) suivi d'un site multiple de clonage et du terminateur de transcription Nos de la nopaline synthase (Figure X+1). La séquence de ce fragment est indiqué (SEQ ID NO 19).

Le vecteur d'expression de l'héliomicine a été obtenu par insertion du fragment de restriction XbaI-KpnI du vecteur pRPA - NP5 contenant la fusion peptide signal PG1 - héliomicine dans le vecteur pILTAB 357 digéré par ces mêmes enzymes. Le clone résultant comporte donc la cassette d'expression promoteur CsVMV - peptide signal PG 1 - héliomicine - terminateur Nos (SEQ ID NO 20).

### Exemple IV-3: Préparation de tabacs transformés.

### 3.1- Transformation

Les vecteurs pRPA-RD-241 ou pRPA-NP6 sont introduits dans la souche *d'Agrobacterium tumefaciens* EHA101 ou EHA105 (Hood & coll., 1987) porteuse du cosmide pTVK291 (Komari & coll., 1986). La technique de transformation est basée sur la procédure de Horsh & coll. (1985).

### 3.2- Régénération

La régénération du tabac PBD6 (provenance SEITA France) à partir d'explants foliaires est réalisée sur un milieu de base Murashige et Skoog (MS) comprenant 30 g/l de saccharose ainsi que 200 µg/ml de kanamycine. Les explants foliaires sont prélevés sur des plantes cultivées en serre ou *in vitro* et régénérées selon la technique des disques foliaires (Horsh & coll., 1985) en trois étapes successives: la première comprend l'induction des pousses sur un milieu additionné de 30 g/l de saccharose contenant 0,05 mg/l d'acide naphtylacétique (ANA) et 2 mg/l de benzylaminopurine (BAP) pendant 15 jours. Les pousses formées au cours de cette étape sont ensuite développées pendant 10 jours par culture sur un milieu MS additionné de 30 g/l de saccharose mais ne contenant pas d'hormone. Puis on prélève des pousses développées et on les cultive sur un milieu d'enracinement MS à teneur moitié en sels, vitamines et sucre et ne contenant pas d'hormone. Au bout d'environ 15 jours, les pousses enracinées sont passées en terre.

### 3.3- Analyse de l'expression de l'héliomicine dans le tabac transgénique

### a) Production d'anticorps polyclonaux spécifiques

Des anticorps polyclonaux ont été obtenus par immunisation d'un lapin avec de l'héliomicine native selon les procédures habituelles du Centre de Bioexpérimentation VALBEX (IUT A - Lyon I). Le serum obtenu (15 ml) a alors été immunopurifié sur colonne de Sepharose 4B (Pharmacia ; ref 17-0430-01) couplée à de l'héliomicine de manière à sélectionner spécifiquement les immunoglobulines reconnaissant ce peptide. Ces anticorps ont été finalement élués dans 6 ml de glycine (200mM ; pH3), neutralisés avec IM Tris pH9,5, dialysés avec 0,5x PBS, et conservés congelés à -20°C jusqu'à utilisation. **b) Immunodétection de l'héliomicine dans le tabac transgénique**

L'analyse de l'expression de l'héliomicine a été conduite sur 8 plantes transgéniques pour la construction pRPA-NP6, ainsi que sur un contrôle sauvage. Des feuilles bien développées de tabac en serre ont été broyées finement à la température de l'azote liquide, et les protéines extraites pendant 1h à 4°C dans le tampon Tris-HCl 50mM, PVP25 1%, 0,05% TritonX100, pH7,5 à raison de 4ml de tampon par gramme de poids frais. Après centrifugation, la concentration en protéines du surnageant a été déterminée par la méthode de Bradford.

Cinq microgrammes de protéines de chacun des 9 extraits ont été déposées sur membrane de nitrocellulose sous un format « slot-blot », ainsi qu'une quantité de 50 ng d'héliomicine pure qui sert de contrôle positif. La membrane a été incubée pendant 1h dans un tampon de bloquage à 1% (Boehringer ; ref 1 921 673) dans du TBS, puis incubée sur la nuit à 4°C avec les anticorps immunopurifiés dirigés contre l'héliomicine dilués au 1/2000 dans le tampon TBS avec 0,05% Tween20. Après lavage (TBS, 0,1 Tween20 et 0,5% de tampon de bloquage), la membrane a été incubée pendant 1h à température ambiante (TBS avec 0,5% tampon de bloquage) avec un anticorps de chèvre (dilué au 1/50000) dirigé spécifiquement contre les immunoglobulines de lapin et couplé à la phosphatase alcaline (SIGMA A-3687). Après lavage (TBS, 0,1% Tween20), la détection se fait en ajoutant un substrat de la phosphatase (BioRad ; ref 170-5012), et la révélation est obtenue par radiographie du produit luminescent sur film Amersham (ECL).

Le résultat de cette expérience montre que 4 plantes de tabac transgénique expriment fortement l'héliomicine. Le signal dans les autres plantes transgéniques est faible ou non significatif par rapport au témoin sauvage. Le signal observé pour la meilleure plante est du niveau du contrôle positif (50 ng d'héliomicine), ce qui indique que dans cette plante l'héliomicine représente pondéralement environ 1% des protéines totales.

### Exemple V-1 : concentrés émulsionnables

### Exemple CE 1 :

- matière active 400 g/l
- dodécylbenzène sulfonate alcalin 24 g/l
- nonylphénol oxyéthylé à 10 molécules d'oxyde d'éthylène 16 g/l
- cyclohexanone 200 g/l
- solvant aromatique q.s.p.1 litre

### Exemple CE 2

- matière active 250 g
- huile végétale époxydée 25 g
- mélange de sulfonate d'alcoylaryle et d'éther de polyglycol et d'alcools gras 100 g
- diAndhylformamide 50 g
- xylène 575 g

### Exemple V-2 : suspension concentrée

### Exemple SC 1 :

- matière active 500 g
- phosphate de tristyrylphénol polyéthoxylé 50 g
- alkylphénol polyéthoxylé 50 g
- polycarboxylate de sodium 20 g
- éthylène glycol 50 g
- huile organopolysiloxanique (antimousse) 1g
- polysaccharide 1,5g
- eau 316,5 g

### Exemple V-3 : poudres mouillables (ou poudres à pulvériser) :

### Exemple PM 1

- matière active 50%
- alcool gras éthoxylé (agent mouillant) 2,5%
- phényléthylphénol éthoxylé (agent dispersant) 5%
- craie (support inerte) 42,5%

### Exemple PM 2 :

- matière active 10%
- alcool synthétique oxo de type ramifié, en C13 éthoxylé par 8 à 10 oxyde d'éthylène (agent mouillant) 0,75%
- lignosulfonate de calcium neutre (agent dispersant) 12%
- carbonate de calcium (charge inerte) q.s.p. 100 %

### Exemple PM 3 :

- matière active 75%
- agent mouillant 1,50%
- agent dispersant 8%
- carbonate de calcium (charge inerte) q.s.p. 100%

### Exemple PM 4 :

- matière active 90%
- alcool gras éthoxylé (agent mouillant) 4%
- phényléthylphénol éthoxylé (agent dispersant) 6%

### Exemple PM 5 :

- matière active 50%
- mélange de tensio-actifs anioniques et non ioniques (agent mouillant) 2,5%
- lignosulfonate de sodium (agent dispersant) 5%
- argile kaolinique (support inerte) 42,5%

### Exemple V-4 : granulés dispersibles

### Exemple GD 1

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

### Exemple GD2

Dans un mélangeur, on mélange les constituants suivants :
- matière active 75%
- agent mouillant (alkylnaphtalène sulfonate de sodium) 2%
- agent dispersant (polynaphtalène sulfonate de sodium) 8%
- charge inerte insoluble dans l'eau (kaolin) 15%

Ce mélange est granulé en lit fluide, en présence d'eau, puis séché, concassé et tamisé de manière à obtenir des granulés de dimension comprise entre 0,15 et 0,80 mm.

### Exemple V-5 : compositions pharmaceutiques

### Exemple A: comprimés

On prépare, selon la technique habituelle, des comprimés dosés à 50 mg de peptide actif ayant la composition suivante:
- peptide héliomicine M1 50 mg
- amidon 60 mg
- lactose 50 mg
- stéarate de magnésium 2 mg

### Exemple B: solution injectable

On prépare une solution injectable contenant 20 mg de peptide actif ayant la composition suivante:
- peptide héliomicine M2 22,4 mg
- eau distillée q.s.p. 2 cm³

### Exemple VI. Stabilité de l'activité de l'héliomicine

La stabilité d'un peptide antimicrobien vis à vis de protéases de plantes est un facteur essentiel pour l'obtention d'un bon niveau d'expression et donc de résistance à des phytopathogènes dans des plantes transgéniques. Cette stabilité est par exemple un point critique pour un peptide antimicrobien d'insecte tel que la cécropine B (Owens et Heutte, 1997. MPM1 vol 10. n°4, pp 525-528). Nous avons examiné la stabilité de l'héliomicine et de son activité sur un phytopathogène test (Botrytis cinerea) après incubation avec des extraits bruts de 8 plantes cultivées majeures (maïs, blé, orge, colza, soja, tournesol, tomate et tabac).

Les feuilles de ces 8 espèces ont été broyées à basse température (azote liquide) dans un mortier, puis la poudre a été resuspendue dans le même volume d'eau. Après centrifugation (10000g pendant 30 minutes), le surnageant a été récupéré, et la concentration en protéine déterminée. Cette concentration a été ajustée pour les 8 extraits à 1mg/ml par dilution avec de l'eau, puis ces extraits ont été filtrés stérilement (0,2 microns). Cent microlitres de chaque extrait (ainsi que un contrôle avec seulement de l'eau) a alors été ajouté à 50 microlitres d'une solution d'héliomicine (contenant 15 microgrammes, ainsi qu'un contrôle sans peptide) dans de l'eau. Ces mélanges ont été incubés à 30°C , un aliquot de 20 microlitres prélevé après 0 h, 1h, 2h, 4h et 20h, immédiatement congelé jusqu'au test.

Le test d'activité antifongique a été réalisé à 25°C en microplaques en ajoutant chaque aliquot à 80 microlitres d'une suspension fraiche de spores de *Botrytis cinerea* (10000 spores/ml dans une solution de Potato Dextrose Broth (Difco, 12g/l). La lecture visuelle des résultats après 12h et 24h montre qu'il n'y a aucune perte significative d'activité antifongique de l'héliomicine, même pour l'échantillon incubé pendant 20h à 30°C, liée à l'exposition à un extrait brut de maïs, blé, orge, colza, soja, tournesol, tomate ou tabac. Ce résultat indique une très forte stabilité de l'héliomicine vis-à-vis de protéases de plantes, et que l'activité antifongique testée sur *Botrytis cinerea* n'est pas affectée par la présence d'extraits bruts végétaux.

### Exemple VII : Réalisation de différents mutants héliomicine: simples, doubles, triples et quadruples mutants.

Les mutants ci-après sont préparés selon la méthode décrite en exemple 11 en remplaçant certains des oligonucléotides 1 à 6 par d'autres oligonucléotides choisis pour introduire les mutations.
- **héliomicine R48:** remplacement de l'acide aminé Glu48 de la séquence ID NO: 1 par un acide aminé basique, en particulier une arginine (Arg48). Par analogie avec la séquence codant pour l'héliomicine de la séquence : SEQ ID NO:1, le codon GAA codant pour Glu est remplacée par le codon AGA codant pour Arg. Les oligonucléotides 19 et 20 sont utilisés en remplacement des oligonucléotides 5 et 6 de l'exemple II.
- **héliomicine L28L29:** remplacement de deux acides aminés basiques Lys et Arg en position 28 et 29 de la séquence ID NO: 1 par deux acides aminés hydrophobes, en particulier deux acides aminés leucines (Leu28 et 29). Par analogie avec la séquence codant pour l'héliomicine de la séquence : SEQ ID NO:1, la partie AAG-CGC codant le reste peptidique Lys-Arg est remplacée par la séquence TTG-TTG codant pour le reste peptidique Leu-Leu. Les oligonucléotides 21 et 22 sont utilisés en remplacement des oligonucléotides 3 et 4 de l'exemple II.
- **héliomicine L28L29R48:** remplacement des deux acides aminés basiques Lys et Arg en position 28 et 29 de la séquence ID NO: 1 par deux résidus acides aminés leucines et remplacement de l'acide aminé Glu48 de la séquence ID NO: 1 par l'acide aminé arginine (Arg48). Les oligonucléotides 19 à 22 sont employés en remplacement des oligonucléotides 3 à 6 selon l'exemple II.
- **héliomicine A24A25:** remplacement des deux acides aminés Asn24 et Gly25 de la séquence ID NO: 1 deux acides aminés alanine (Ala24 et Ala25). Par analogie avec la séquence codant pour l'héliomicine de la séquence ID NO:1, la partie AAC-GGC codant le reste peptidique Asn-Gly est remplacée par la séquence GCT-GCT codant pour Ala-Ala. Les oligonucléotides 23 et 24 sont utilisés en remplacement des oligonucléotides 3 et 4 de l'exemple II.
- **héliomicine A6A7A8A9:** remplacement des acides aminés Asp6-Lys7-Leu8-Ile9 de la séquence ID NO: 1 par 4 acides aminés alanine (Ala). Par analogie avec la séquence codant pour l'héliomicine de la séquence ID NO:1, la partie GAC-AAG-TTG-ATT codant le reste peptidique Asp-Lys-Leu-Ile est remplacée par la séquence GCT-GCT-GCT-GCT codant pour le reste peptidique Ala-Ala-Ala-Ala. Les oligonucléotides 25 et 26 sont utilisés en remplacement de l'oligonucléotide 1 de l'exemple II et les oligonucléotides 27 et 28 en remplacement de l'oligonucléotide 2.
- héliomicine A24A25L28L29: Deux oligonucléotides (sens et antisens) ont été nécessaires pour palier à l'absence d'un site de restriction entre la séquence codant pour le reste peptidique constitué des deux acides aminés Asn24-Gly25 et la séquence codant pour le reste peptidique constitué des deux acides aminés Lys28-Arg29 de l'héliomicine de la séquence ID NO: 1. Les deux séquences oligonucléotidiques 29 et 30 remplacent respectivement les deux séquences oligonucléotidiques 3 et 4 de l'exemple II.

### Production d'héliomicine mutée à l'échelle semi-préparative.

Les différents mutants d'héliomicine sont préparés et purifiés de la façon suivante. Un des clones de levure transformée exprimant l'héliomicine mutée a été cultivé à 29°C pendant 48 h dans 50 ml de milieu sélectif. Cette préculture a ensuite été utilisée pour inoculer 2 1 de milieu sélectif et la culture a été réalisée pendant 48 h à 29 °C. Les levures ont été éliminées par centrifugation (4000 g, 30 min, 4°C). Le surnageant a été acidifié jusqu'à pH 3,5 avec de l'acide acétique, soumis à une deuxième centrifugation (4000 g, 30 min, 4°C) avant une première étape d'extraction en phase solide.
- première étape d'extraction en phase solide sur un gel de phase inverse : le surnageant acidifié est déposé sur une cartouche Sep-Pak Vac 35cc de phase inverse C18 (Waters Associates, 10 g de phase) équilibrée avec de l'eau acidifiée (TFA 0,05 %). Les molécules hydrophiles ont été éliminées par un lavage avec de l'eau acidifiée suivie d'un lavage avec une solution d'acétonitrile à 15% préparée dans le TFA 0,05%. L'élution du peptide a été réalisée par une solution d'acétonitrile à 60% préparée dans le TFA 0,05%. La fraction éluée à 60% d'acétonitrile a été lyophilisée puis reconstituée dans de l'eau ultrapure stérile avant d'être soumise à la première étape de purification.
- deuxième étape d'extraction en phase solide sur un gel échangeur de cations : la fraction 60% prépurifiée contenant l'héliomicine mutée a été reconstituée dans une solution d'acétate d'ammonium à 25 mM, pH 3,4. Cet échantillon a été déposée sur une cartouche Sep-Pak Vac 35cc CM échangeuse de cations (Waters Associates, 10 g de phase) équilibrée avec de l'acétate d'ammonium à 25 mM, pH 3,4. L'héliomicine mutée est éluée en utilisant une solution à 1 M de chlorure de sodium (NaCl) préparée dans l'acétate d'ammonium à 25 mM, pH 3.4. La fraction 1 M NaCl contenant l'héliomicine mutée est recueillie, asséchée sous vide, reconstituée avec 20 ml d'eau ultrapure acidifiée (TFA 1 %). L'héliomicine mutée est alors purifiée par HPLC de phase inverse.
- dernière étape de purification par HPLC : l'héliomicine mutée a été purifiée jusqu'à homogénéité par chromatographie sur une colonne de phase inverse préparative Aquapo re RP-300 C8 (Brownlee™, 220 x 10 mm, 300 Å), en utilisant un gradient linéaire diphasique d'acétonitrile de 2% à 23% en 10 min et de 23% à 33% en 80 min dans le TFA 0,05% au débit constant de 2,5 ml/min. La fraction recueillie est asséchée sous vide, reconstituée avec de l'eau ultrapure et analysée par spectrométrie de masse MALDI pour en vérifier la pureté et l'identité. Les différentes héliomicines mutées ont été analysées pour leur activité antifongique dans les conditions décrites pour l'héliomicine de référence contre les souches suivantes : Neurospora crassa, Fusarium culmorum et Nectria haematococca. L'activité des mutants d'héliomicine a également été évaluée contre des bactéries. Les conditions expérimentales utilisées sont décrites ci-dessous.

### Test d'activité in vitro : mesure de l'activité antibactérienne et antifongique par microspectrophotométrie.

Cette méthodologie a été utilisée pour la détermination du spectre d'activité du peptide et de la concentration minimale inhibitrice (CMI) à laquelle le peptide muté est actif. La CMI a été exprimée comme un intervalle de concentration [a] - [b] où [a] a été la concentration minimale où l'on observe un début de croissance et [b] la concentration pour laquelle aucune croissance n'a été observée. Des exemples de l'activité spécifique de l'héliomicine mutée, vis-à-vis des bactéries et champignons filamenteux sont donnés dans le tableau 3.

L'activité antibactérienne a été détectée par un test d'inhibition de croissance en milieu liquide. Les bactéries à tester ont été mises en suspension dans un milieu nutritif de type " Poor Broth ". De préférence, en utilise une solution de bactotryptone à 1% additionnée de NaCl à 1% en poids volume préparée dans de l'eau déminéralisée. On dépose 10 µl de chaque fraction a analyser dans des plaques de microtitration en présence de 90 µl de milieu de culture contenant les bactéries (à une concentration finale équivalente à 1mDO à 600 nm). L'incubation a été réaliser à 25°C durant 12 à 24 heures. La croissance bactérienne a été mesurée en suivant l'absorbance à 600 nm à l'aide d'un spectrophotomètre lecteur de plaque de microtitration.
- bactéries testées : *Bacillus megaterium* (collection de Institut Pasteur). *Micrococcus luteus* (collection de l'Institut Pasteur). *Staphylococcus aureus* (H. Monteil, Institut de bactériologie, Strasbourg), *Aerococcus viridans* (H. Monteil, Institut de bactériologie, Strasbourg), et *Escherichia coli D22* (P. L. Boquet, Centre d'Etudes Nucléaires, Saclay).

**Tableau 3 : activité de certaines héliomicines mutées contre les champignons filamenteux et les bactéries**

| Micro-organismes | CMI des mutants d'héliomicine (µM) | | | | |
|---|---|---|---|---|---|
| | L28L29 | R48 | L28L29R48 | A6A7A8A9 | Hélio |
| Champignons | | | | | |
| *Neurospora crassa* | 0,8-1,6 | 0,4-0,8 | 0,8-1,6 | 1,6-3,1 | 0,1-0,2 |
| *Fusarium culmorum* | 3,1-6,2 | 0,4-0,8 | 0,8-1,6 | 3,1-6,2 | 0,2-0,4 |
| Nectria haematococca | 3,1-6,2 | 0,4-0,8 | 0,8-1,6 | ND | 0,4-0,8 |
| Bactéries | | | | | |
| *Bacillus megaterium* | 50-100 | ND | ND | 6,2-12,5 | ND |
| *Micrococcus luteus* | 12,5-25 | 25-50 | ND | ND | ND |
| *Staphyloccoccus aureus* | ND | ND | ND | ND | ND |
| *Aerococcus viridans* | ND | ND | ND | 12.5-25 | ND |
| *Escherichia coli D22* | ND | ND | ND | ND | ND |

| | | | | | |
|---|---|---|---|---|---|
| ND: pas d'activité détectée. | | | | | |

### Exemple VIII : Etude de toxicité aiguë

Des groupes de 4 souris femelles ont été traitées par injection intraveineuse de solutions d'héliomicine (SEQ ID NO 2) en solution saline à des doses de 1 et 10 mg/kg. Des solutions correspondantes d'aprotinine comme contrôle négatif (pas d'effets pour les deux doses) et de mellitine comme contrôle positif (100% de mortalité à 5 jours à 10 mg, effets significatifs à 5 jours à 1mg). Aucune toxicité n'a été mise en évidence pour les solutions d'héliomicine aux deux doses injectées.

### REFERENCES

Ausubel, F. A. & coll. (eds. Greene). Current Protocols in Molecular Biology. Publ. Wiley & Sons.
Bevan. M. & coll. (1983). Nuc. Acids Res. 11:369-385.
Carrington and Freed (1990). J. Virol. 64:1590-1597.
Ehret-Sabatier & coll. (1996) The Journal of Biological Chemistry, 271, 47, 29537-29544. Horsch & coll. (1985). Science 227:1229-1231.
Jefferson & coll. (1987). EMBO J. 6:3901-3907.
Komari & coll. (1986). J. Bacteriol. 166:88-94.
Rothstein & coll. (1981). Cold Spring Harb. Symp. Quant. Biol. 45: 99-105.
Stalker & coll. (1988). J. Biol. Chem. 263:6310-6314.
Odell, J.T. & coll. (1985). Nature 313:810-812.

## Revendications

1. Peptide comprenant la séquence peptidique de formule (I),
Xaa-Cys-Xab-Cys-Xac-Cys-Xad-Cys-Xae-Cys-Xaf-Cys-Xag (I)
dans laquelle:
Xaa est -NH₂ ou un reste peptidique comprenant de 1 à 10 acides aminés, de préférence de 1 à 6 acides aminés, comprenant au moins un acide aminé basique, représentant la séquence peptidique suivante Xaa'-Gly-Xaa"- dans laquelle Xaa' représente NH2 ou un reste peptidique comprenant 1 à 9 acides aminés, et Xaa" représente un reste peptidique comprenant un acide aminé, choisi parmi Leu, Ile; Val; Pro, Ser ou Thr,
Xab est un reste peptidique comprenant 10 acides aminés,
Xac est un reste peptidique comprenant 3 acides aminés, comprenant au moins un acide aminé acide,
Xad représente la séquence peptidique suivante -Lys-Arg-Arg-Gly-Tyr-Lys-Gly-Gly-His-
Xae représente la séquence peptidique suivante -Gly-Xae'-Asn-, dans laquelle Xae' représente un reste peptidique comprenant 5 acides aminés,
Xaf est un tryptophane, et
Xag est -OH ou un reste peptidique comprenant de 1 à 2 acides aminés.

2. Peptide selon la revendication 1, **caractérisé en ce que** les acides aminés basiques sont choisis parmi la lysine, l'arginine ou l'homoarginine.

3. Peptide selon l'une des revendications 1 ou 2, **caractérisé en ce que** Xac représente la séquence peptidique suivante -Asn-Xac'-Xac"-, dans laquelle Xac' représente un reste peptidique de 1 acide aminé, et Xac" représente un reste peptidique de 1 acide aminé acide.

4. Peptide selon l'une des revendications 1 à 3, **caractérisé en ce que** lesdits acides aminés acides sont choisis parmi l'acide glutamique (Glu) ou l'acide aspartique (Asp).

5. Peptide selon l'une des revendications 1 à 4, **caractérisé en ce que** Xac représente la séquence peptidique suivante -Asn-Gly-Glu-.

6. Peptide selon l'une des revendications 1 à 5, **caractérisé en ce que** Xab représente la séquence peptidique suivante -Val-Xab'-Asp-, dans laquelle Xab' représente un reste peptidique comprenant 8 acides aminés, et/ou Xag représente la séquence peptidique suivante -Glu-Xag' dans laquelle Xag' représente OH ou un reste variable de séquence comprenant 1 acide aminé.

7. Peptide selon l'une des revendications 1 à 6, **caractérisé en ce que** Xaa représente la séquence peptidique suivante NH₂-Asp-Lys-Leu-Ile-Gly-Ser-, et/ou
Xab représente la séquence peptidique suivante -Val-Trp-Gly-Ala-Val-Asn-Tyr-Thr-Ser-Asp-, et/ou
Xae représente la séquence peptidique suivante -Gly-Ser-Phe-Ala-Asn-Val-Asn-, et/ou
Xag représente la séquence peptidique suivante -Glu-Thr-OH.

8. Peptide selon l'une des revendications 1 à 7, **caractérisé en ce qu'**il est représenté par l'identificateur de n° 2 (SEQ ID NO 2).

9. Peptide selon l'une des revendications 1 à 8, **caractérisé en ce qu'**il comprend à l'une ou l'autre de ses extrémités, ou les deux, des résidus peptidiques nécessaires à son expression et ciblage dans un organisme hôte.

10. Peptide selon l'une des revendications 1 à 9, **caractérisé en ce que** les résidus cystéines du peptide de formule (I) forment au moins un pont disulfure intramoléculaire.

11. Peptide selon la revendication 10, **caractérisé en ce qu'**il comprend 3 ponts disulfures établis entre les résidus cystéine 1 et 4, 2 et 5 et 3 et 6.

12. Peptide de fusion «peptide-héliomicine», comprenant un peptide héliomicine selon l'une des revendications 1 à 11, et un reste peptidique susceptible d'être clivé par des systèmes enzymatiques d'un organisme hôte, permettant la libération de l'héliomicine.

13. Peptide de fusion selon la revendication 12, **caractérisé en ce que** le peptide fusionné à l'héliomicine est un peptide signal ou un peptide de transit.

14. Peptide de fusion selon la revendication 13, **caractérisé en ce que** le peptide de transit est le peptide signal du gène PR-1α du tabac, le précurseur du facteur Mat alpha 1 ou le peptide signal du gène PG1 de polygalacturonase de maïs.

15. Peptide de fusion selon la revendication 14, **caractérisé en ce qu'**il est représenté par l'identificateur de séquence n° 1 (SEQ ID NO 1), par l'identificateur de séquence n° 3 (SEQ ID No 3), ou par l'identificateur de séquence n° 18 (SEQ ID NO 18).

16. A titre de médicament, le peptide selon l'une des revendications 1 à 15.

17. Composition **caractérisée en ce qu'**elle comprend le peptide selon l'une des revendications 1 à 15 et un véhicule approprié.

18. Fragment d'acide nucléique, **caractérisé en ce qu'**il comprend une séquence d'acide nucléique codant pour un peptide selon l'une des revendications 1 à 15.

19. Fragment d'acide nucléique selon la revendication 18, **caractérisé en ce qu'**il s'agit d'une séquence nucléotidique de type ADN.

20. Fragment d'acide nucléique selon la revendication 19, **caractérisé en ce que** la séquence nucléotidique de type ADN comprend la séquence d'ADN décrite par les bases 16 à 147 de l'identificateur de séquence n° 1 (SEQ ID NO 1), par l'identificateur de séquence n° 2 (SEQ ID NO 2), par les bases 3 à 224 de l'identificateur se séquence n° 3 (SEQ ID NO 3), ou par les bases 7 à 205 de l'identificateur de séquence n° 18 (SEQ ID NO 18), une séquence homologue ou une séquence complémentaire de ladite séquence.

21. Gène chimère comprenant une séquence codante ainsi que des éléments de régulation en position 5' et 3' hétérologues pouvant fonctionner dans un organisme hôte, en particulier les plantes, **caractérisé en ce que** la séquence codante comprend au moins un fragment d'ADN tel que défini dans les revendications 18 à 20.

22. Gène chimère selon la revendication 21, **caractérisé en ce que** l'organisme hôte est un microorganisme.

23. Gène chimère selon la revendication 21, **caractérisé en ce que** l'organisme hôte est choisi parmi les cellules végétales et les plantes.

24. Vecteur de clonage ou d'expression pour la transformation d'un organisme hôte **caractérisé en ce qu'**il comprend au moins une origine de réplication et au moins un gène chimère tel que défini dans les revendications 21 à 23.

25. Organisme hôte non humain transformé, **caractérisés en ce qu'**ils contient un fragment d'acide nucléique selon l'une des revendications 18 à 20, ou un gène chimère selon l'une des revendications 21 à 23.

26. Organisme hôte transformé selon la revendication 25, **caractérisé en ce qu'**il s'agit d'un micro organisme, d'une cellule végétale ou d'une plante.

27. Organisme hôte transformé selon la revendication 26, **caractérisé en ce qu'**il s'agit d'une plante contenant des cellules transformées.

28. Organisme hôte selon la revendication 27, **caractérisé en ce que** la plante est régénérée à partir des cellules transformées.

29. Organisme hôte transformé selon la revendication 26, **caractérisé en ce que** le microorganisme est choisi parmi les bactéries, en particulier *E. coli,* les levures, en particulier des genres *Saccharomyces* ou *Kluyveromyces, Pichia,* les champignons, en particulier *Aspergillus,* ou les bacilovirus,

30. Cellule végétale transformée, **caractérisée en ce qu'**elle contient un fragment d'acide nucléique selon l'une des revendications 18 à 20 ou un gène chimère selon l'une des revendications 21 à 23.

31. Plante transformée, **caractérisée en ce qu'**elle comprend au moins une cellule végétale transformée selon la revendication 30.

32. Plante transformée selon la revendication 31, **caractérisée en ce qu'**elle est résistante aux maladies causées par *Cercospora,* en particulier *Cercospora beticola, Cladosporium* en particulier *Cladosporium herbarum, Fusarium,* en particulier *Fusarium culmorum* ou *Fusarium graminearum,* ou par *Phytophthora,* en particulier *Phytophtora cinnamomi.*

33. Plante transformées **caractérisée en ce qu'**elle est issue de la culture et/ou du croisement des plantes selon l'une des revendications 31 ou 32, **et ce qu'elle contient un fragment d'acide nucléique selon l'une des revendications 18 à 20 ou un gène chimère selon l'une des revendications 21 à 23.**

34. Graines de plantes transformées selon l'une des revendications 31 à 33, **contenant un fragment d'acide nucléique selon l'une des revendications 18 à 20 ou un gène chimère selon l'une des revendications 21 à 23.**

35. Procédé de transformation des organismes hôtes non humains, en particulier des cellules végétales ou des plantes, **caractérisé en ce que** l'on insère dans ledit organisme hôte au moins un fragment d'acide nucléique selon l'une des revendications 18 à 20 ou un gène chimère selon l'une des revendications 21 à 23.

36. Procédé selon la revendication 35, **caractérisé en ce que** l'organisme hôte est une cellule végétale ou une plante.

37. Procédé selon la revendication 36, **caractérisé en ce que** l'on régénère une plante à partir de la cellule végétale ou de la plante transformée.

38. Procédé de culture des plantes transformées selon l'une des revendications 31 à 33, **caractérisé en ce qu'**il consiste à planter les graines des dites plantes transformées dans une surface d'un champ approprié pour la culture des dites plantes, à appliquer sur la dite surface du dit champ une composition agrochimique, sans affecter de manière substantielle les dites graines ou les dites plantes transformées, puis à récolter les plantes cultivées lorsqu'elles arrivent à la maturité souhaitée et éventuellement à séparer les graines des plantes récoltées.

39. Procédé de culture selon la revendication 38, **caractérisé en ce que** la composition agrochimique comprend au moins un produit actif ayant au moins une activité fongicide et/ou bactéricide.

40. Procédé de culture selon la revendication 39, **caractérisé en ce que** le produit actif présente une activité complémentaire de celle du peptide selon l'une des revendications 1 à 15.

41. Procédé de préparation d'héliomicine définie selon l'une des revendications 1 à 15, **caractérisé en ce qu'**il comprend comprenant les étapes de culture d'un organisme transformé selon l'une des revendications 25 à 29, dans un milieu de culture approprié, puis l'extraction et la purification totale ou partielle de l'héliomicine obtenue.

## Claims

1. Peptide comprising the peptide sequence of formula (I),
Xaa-Cys-Xab-Cys-Xac-Cys-Xad-Cys-Xae-Cys-Xaf-Cys-Xag (I)
in which:
Xaa is -NH2 or a peptide residue comprising from 1 to 10 amino acids, preferably from 1 to 6 amino acids, comprising at least one basic amino acid, representing the following peptide sequence Xaa'-Gly-Xaa"- in which Xaa' represents NH2 or a peptide residue comprising 1 to 9 amino acids, and Xaa" represents a peptide residue comprising one amino acid, chosen from Leu, Ile, Val, Pro, Ser or Thr,
Xab is a peptide residue comprising 10 amino acids,
Xac is a peptide residue of 3 amino acids, comprising at least one acidic amino acid,
Xad represents the following peptide sequence -Lys-Arg-Arg-Gly-Tyr-Lys-Gly-Gly-His-,
Xae represents the following peptide sequence -Gly-Xae'-Asn-, in which Xae' represents a peptide residue comprising 5 amino acids,
Xaf is a tryptophane, and
Xag is -OH or a peptide residue comprising from 1 to 2 amino acids.

2. Peptide according to claim 1, **characterized in that** the basic amino acids are chosen from lysine, arginine or homoarginine.

3. Peptide according to one of claims 1 or 2, **characterized in that** Xac represents the following peptide sequence -Asn-Xac'-Xac"-, in which Xac' represents a peptide residue of 1 amino acid, and Xac" represents a peptide residue of 1 acidic amino acid.

4. Peptide according to one of claims 1 to 3, **characterized in that** the acidic amino acids are chosen from glutamic acid (Glu) or aspartic acid (Asp).

5. Peptide according to one of claims 1 to 4, **characterized in that** Xac represents the following peptide sequence -Asn-Gly-Glu-.

6. Peptide according to one of claims 1 to 5, **characterized in that**
Xab represents the following peptide sequence -Val-Xab'-Asp-, in which Xab' represents a peptide residue comprising 8 amino acids, and/or
Xag represents the following peptide sequence -Glu-Xag' in which Xag' represents OH or a variable residue having a sequence comprising 1 amino acid.

7. Peptide according to one of claims 1 to 6, **characterized in that**
Xaa represents the following peptide sequence NH2-Asp-Lys-Leu-Ile-Gly-Ser-, and/or
Xab represents the following peptide sequence -Val-Trp-Gly-Ala-Val-Asn-Tyr-Thr-Ser-Asp-, and/or
Xae represents the following peptide sequence -Gly-Ser-Phe-Ala-Asn-Val-Asn-, and/or
Xag represents the following peptide sequence -Glu-Thr-OH.

8. Peptide according to one of claims 1 to 7, **characterized in that** it is represented by the identifier No. 2 (SEQ ID NO 2).

9. Peptide according to one of claims 1 to 8, **characterized in that** it comprises at either of its ends, or at both ends, peptide residues necessary for its expression and targeting in a host organism.

10. Peptide according to one of claims 1 to 9, **characterized in that** the cysteine residues of the peptide of formula (I) form at least one intramolecular disulphide bridge.

11. Peptide according to claim 10, **characterized in that** it comprises 3 disulphide bridges established between the cysteine residues 1 and 4, 2 and 5, and 3 and 6.

12. "Peptide-heliomicine" fusion peptide, comprising a heliomicine peptide according to one of claims 1 to 11, and a peptide residue capable of being cleaved by the enzymatic systems of a host cell, allowing release of the heliomicine.

13. Fusion peptide according to claim 12, **characterized in that** the peptide fused with heliomicine is a signal peptide or a transit peptide.

14. Fusion peptide according to claim 13, **characterized in that** the transit peptide is the signal peptide of the tobacco PR-1α gene, the precursor of factor Mat alpha 1 or the signal peptide of the maize polygalacturonase PG1 gene.

15. Fusion peptide according to claim 14, **characterized in that** it is represented by the sequence identifier No. 1 (SEQ ID NO 1), by the sequence identifier No. 3 (SEQ ID NO 3), or by the sequence identifier No. 18 (SEQ ID NO 18).

16. As a medicament, the peptide according to one of claims 1 to 15.

17. Composition, **characterized in that** it comprises the peptide according to one of claims 1 to 15 and an appropriate vehicle.

18. Nucleic acid fragment, **characterized in that** it comprises a nucleic acid sequence encoding a peptide according to one of claims 1 to 15.

19. Nucleic acid fragment according to claim 18, **characterized in that** it is a nucleotide sequence of the DNA type.

20. Nucleic acid fragment according to claim 19, **characterized in that** the nucleotide sequence of the DNA type comprises the DNA sequence described by bases 16 to 147 of the sequence identifier No. 1 (SEQ ID NO 1), by the sequence identifier No. 2 (SEQ ID NO 2), by bases 3 to 224 of the sequence identifier No. 3 (SEQ ID NO 3), or by bases 7 to 205 of the sequence identifier No. 18 (SEQ ID NO 18), a homologous sequence or a sequence complementary to the said sequence.

21. Chimeric gene comprising a coding sequence as well as heterologous regulatory elements at the 5' and 3' positions capable of functioning in a host organism, in particular plants, **characterized in that** the coding sequence comprises at least one DNA fragment as defined in claims 18 to 20.

22. Chimeric gene according to claim 21, **characterized in that** the host organism is a microorganism.

23. Chimeric gene according to claim 21, **characterized in that** the host organism is chosen from plant cells and plants.

24. Cloning or expression vector for the transformation of a host organism, **characterized in that** it comprises at least one replication origin and at least one chimeric gene as defined in claims 21 to 23.

25. Transformed non-human host organism, **characterized in that** it contains a nucleic acid fragment according to one of claims 18 to 20, or a chimeric gene according to one of claims 21 to 23.

26. Transformed host organism according to claim 25, **characterized in that** it includes microorganisms, plant cells or plants.

27. Transformed host organism according to claim 26, **characterized in that** it is a plant containing transformed cells.

28. Host organism according to claim 27, **characterized in that** the plant is regenerated from transformed cells.

29. Transformed host organism according to claim 30, **characterized in that** the microorganism is chosen from bacteria, in particular *E. coli,* yeasts, in particular of the genera *Saccharomyces* or *Kluyveromyces, Pichia,* fungi, in particular *Aspergillus,* or baculoviruses.

30. Transformed plant cell, **characterized in that** it contains a nucleic acid fragment according to claims one of 18 to 20 or a chimeric gene according to one of claims 21 to 23.

31. Transformed plant, **characterized in that** it comprises at least one transformed plant cell according to claim 30.

32. Transformed plant according to claim 31, **characterized in that** it is resistant to diseases caused by *Cercospora,* in particular *Cercospora beticola, Cladosporium,* in particular *Cladosporium herbarum, Fusarium,* in particular *Fusarium culmorum* or *Fusarium graminearum,* or by *Phytophthora,* in particular *Phytophthora cinnamomi.*

33. Transformed plant, **characterized in that** it is derived from the cultivation and/or crossing of the plants according to either of claims 31 and 32, and that it contains a nucleic acid fragment according to one of claims 18 to 20 or a chimeric gene according to one of claims 21 to 23.

34. Seeds of transformed plants according to one of claims 31 to 33, containing a nucleic acid fragment according to one of claims 18 to 20 or a chimeric gene according to one of claims 21 to 23.

35. Method of transforming non-human host organisms, in particular plant cells or plants, **characterized in that** at least one nucleic acid fragment according to one of claims 18 to 20 or a chimeric gene according to one of claims 21 to 23 is inserted into the said host organism.

36. Method according to claim 35, **characterized in that** the host organism is a plant cell or a plant.

37. Method according to claim 36, **characterized in that** a plant is regenerated from the plant cell or from the transformed plant.

38. Method of cultivating transformed plants according to one of claims 31 to 33, **characterized in that** it consists in planting the seeds of the said transformed plants in a plot of a field appropriate for cultivating the said plants, in applying to the said plot of the said field an agrochemical composition, without substantially affecting the said seeds or the said transformed plants, then in harvesting the cultivated plants when they arrive at the desired maturity and optionally in separating the seeds from the harvested plants.

39. Method of cultivation according to claim 38, **characterized in that** the agrochemical composition comprises at least one active product having at least one fungicidal and/or bactericidal activity.

40. Method of cultivation according to claim 39, **characterized in that** the active product exhibits an activity which is complementary to that of the peptide according to one of claims 1 to 15.

41. Method of preparing heliomicine defined according to one of claims 1 to 15, **characterized in that** it comprises the steps of culturing a transformed organism according to one of claims 25 to 29 in an appropriate culture medium, followed by the extraction and total or partial purification of the heliomicine obtained.

## Patentansprüche

1. Peptid umfassend die Peptidsequenz der Formel (I),
Xaa-Cys-Xab-Cys-Xac-Cys-Xad-Cys-Xae-Cys-Xaf-Cys-Xag (I)
in der:
Xaa -NH₂ oder ein Peptidrest ist, umfassend von 1 bis 10 Aminosäuren, bevorzugt von 1 bis 6 Aminosäuren, umfassend mindestens eine basische Aminosäure, der die nachstehende Peptidsequenz Xaa'-Gly-Xaa"- darstellt, in der Xaa' NH₂ oder einen Peptidrest umfassend 1 bis 9 Aminosäuren darstellt, und Xaa" einen Peptidrest umfassend eine Aminosäure ausgewählt aus Leu, Ile, Val, Pro, Ser oder Thr darstellt,
Xab ein Peptidrest umfassend 10 Aminosäuren ist,
Xac ein Peptidrest umfassend 3 Aminosäuren, umfassend mindestens eine saure Aminosäure ist,
Xad die nachstehende Peptidsequenz -Lys-Arg-Arg-Gly-Tyr-Lys-Gly-Gly-His- darstellt,
Xae die nachstehende Peptidsequenz -Gly-Xae'-Asn- darstellt, in der Xae' einen Peptidrest umfassend 5 Aminosäuren darstellt,
Xaf ein Tryptophan ist, und
Xag -OH oder ein Peptidrest umfassend von 1 bis 2 Aminosäuren ist.

2. Peptid nach Anspruch 1, **dadurch gekennzeichnet, dass** die basischen Aminosäuren aus Lysin, Arginin, oder Homoarginin ausgewählt sind.

3. Peptid nach einem der Ansprüche 1 oder 2, **dadurch gekennzeichnet, dass** Xac die nachstehende Peptidsequenz -Asn-Xac'-Xac"- darstellt, in der Xac' einen Peptidrest aus 1 Aminosäure darstellt und Xac" einen Peptidrest aus 1 Aminosäure darstellt.

4. Peptid nach einem der Ansprüche 1 bis 3, **dadurch gekennzeichnet, dass** die besagten Aminosäuren aus Glutaminsäure (Glu) oder Asparaginsäure (Asp) ausgewählt sind.

5. Peptid nach einem der Ansprüche 1 bis 4, **dadurch gekennzeichnet, dass** Xac die nachstehende Peptidsequenz -Asn-Gly-Glu- darstellt.

6. Peptid nach einem der Ansprüche 1 bis 5, **dadurch gekennzeichnet, dass** Xab die nachstehende Peptidsequenz -Val-Xab'-Asp- darstellt, in der Xab' einen Peptidrest umfassend 8 Aminosäuren darstellt, und/oder Xag die nachstehende Peptidsequenz -Glu-Xag' darstellt, in der Xag' OH oder einen variablen Rest einer Sequenz umfassend 1 Aminosäure darstellt.

7. Peptid nach einem der Ansprüche 1 bis 6, **dadurch gekennzeichnet, dass**
Xaa die nachstehende Peptidsequenz NH₂-Asp-Lys-Leu-Ile-Gly-Ser- darstellt, und/oder
Xab die nachstehende Peptidsequenz -Val-Trp-Gly-Ala-Val-Asn-Tyr-Thr-Ser-Aspdarstellt, und/oder
Xae die nachstehende Peptidsequenz -Gly-Ser-Phe-Ala-Asn-Val-Asn- darstellt, und/oder
Xag die nachstehende Peptidsequenz -Glu-Thr-OH darstellt.

8. Peptid nach einem der Ansprüche 1 bis 7, **dadurch gekennzeichnet, dass** es durch den Identifikator Nr. 2 dargestellt ist (SEQ ID Nr. 2).

9. Peptid nach einem der Ansprüche 1 bis 8, **dadurch gekennzeichnet, dass** es an dem einen oder anderen, oder beiden seiner Enden Peptidreste umfasst, die zu seiner Expression und dem zielgerichteten Transport in einen Wirtsorganismus notwendig sind.

10. Peptid nach einem der Ansprüche 1 bis 9, **dadurch gekennzeichnet, dass** die Cysteinreste des Peptids der Formel (I) mindestens eine intramolekulare Disulfidbrücke bilden.

11. Peptid nach Anspruch 10, **dadurch gekennzeichnet, dass** es drei Disulfidbrücken umfasst, die zwischen den Cysteinresten 1 und 4, 2 und 5, und 3 und 6 ausgebildet sind.

12. Fusionspeptid "Peptid-Heliomycin", umfassend ein Heliomycinpeptid nach einem der Ansprüche 1 bis 11 und einen Peptidrest, der die Fähigkeit besitzt, durch die Enzymsysteme eines Wirtsorganismus gespalten zu werden, wodurch die Freisetzung des Heliomycins ermöglicht wird.

13. Fusionspeptid nach Anspruch 12, **dadurch gekennzeichnet, dass** das an das Heliomycin fusionierte Peptid ein Signalpeptid oder ein Transitpeptid ist.

14. Fusionspeptid nach Anspruch 13, **dadurch gekennzeichnet, dass** das Transitpeptid das Signalpeptid des PR-1α-Gens aus Tabak, der Vorläufer des Faktors Mat-alpha-1, oder das Signalpeptid des Gens PG1 von Polygalacturonase aus Mais ist.

15. Fusionspeptid nach Anspruch 14, **dadurch gekennzeichnet, dass** es durch den Identifikator der Sequenz Nr. 1 (SEQ ID Nr. 1), durch den Identifikator der Sequenz Nr. 3 (SEQ ID Nr. 3), oder durch den Identifikator der Sequenz Nr. 18 (SEQ ID Nr. 18) dargestellt wird.

16. Peptid nach einem der Ansprüche 1 bis 15 als Medikament.

17. Zusammensetzung, **dadurch gekennzeichnet, dass** sie das Peptid nach einem der Ansprüche 1 bis 15 und ein geeignetes Trägermaterial umfasst.

18. Nukleinsäurefragment, **dadurch gekennzeichnet, dass** es eine Nukleinsäuresequenz umfasst, die für ein Peptid nach einem der Ansprüche 1 bis 15 kodiert.

19. Nukleinsäurefragment nach Anspruch 18, **dadurch gekennzeichnet, dass** es sich um eine Nukleotidsequenz vom DNA-Typ handelt.

20. Nukleinsäurefragment nach Anspruch 19, **dadurch gekennzeichnet, dass** die Nukleotidsequenz vom DNA-Typ die DNA-Sequenz umfasst, die durch die Basen 16 bis 147 des Identifikators der Sequenz Nr. 1 (SEQ ID Nr. 1), durch den Identifikator der Sequenz Nr. 2 (SEQ ID Nr. 2), durch die Basen 3 bis 224 des Identifikators der Sequenz Nr. 3 (SEQ ID Nr. 3) oder durch die Basen 7 bis 205 des Identifikators der Sequenz Nr. 18 (SEQ ID Nr. 18), einer homologen Sequenz oder einer zur besagten Sequenz komplementären Sequenz beschrieben wird.

21. Chimäres Gen, umfassend eine kodierende Sequenz sowie heterologe Regulationselemente in Position 5' und 3', die in einem Wirtsorganismus funktionsfähig sein können, insbe-sondere in Pflanzen, **dadurch gekennzeichnet, dass** die kodierende Sequenz mindestens ein DNA-Fragment wie in den Ansprüchen 18 bis 20 definiert umfasst.

22. Chimäres Gen nach Anspruch 21, **dadurch gekennzeichnet, dass** der Wirtsorganismus ein Mikroorganismus ist.

23. Chimäres Gen nach Anspruch 21, **dadurch gekennzeichnet, dass** der Wirtsorganismus aus pflanzlichen Zellen und Pflanzen ausgewählt ist.

24. Klonierungs- oder Expressionsvektor zur Transformation eines Wirtsorganismus, **dadurch gekennzeichnet, dass** er mindestens einen Replikationsursprung und mindestens ein chimäres Gen, wie in den Ansprüchen 21 bis 23 definiert, umfasst.

25. Transformierter nicht-menschlicher Wirtsorganismus, **dadurch gekennzeichnet, dass** er ein Nukleinsäure-fragment nach einem der Ansprüche 18 bis 20, oder ein chimäres Gen nach einem der Ansprüche 21 bis 23 enthält.

26. Transformierter Wirtsorganismus nach Anspruch 25, **dadurch gekennzeichnet, dass** es sich um einen Mikroorganismus, eine pflanzliche Zelle oder eine Pflanze handelt.

27. Transformierter Wirtsorganismus nach Anspruch 26, **dadurch gekennzeichnet, dass** es sich um eine Pflanze, die transformierte Zellen enthält, handelt.

28. Wirtsorganismus nach Anspruch 27, **dadurch gekennzeichnet, dass** die Pflanze aus transformierten Zellen regeneriert wird.

29. Transformierter Wirtsorganismus nach Anspruch 26, **dadurch gekennzeichnet, dass** der Mikroorganismus aus Bakterien, insbesondere *E. coli,* Hefen, insbesondere der Gattungen *Saccharomyces* oder *Kluyveromyces, Pichia,* Pilzen, insbesondere *Aspergillus,* oder Baculoviren ausgewählt ist.

30. Transformierte pflanzliche Zelle, **dadurch gekennzeichnet, dass** sie ein Nukleinsäurefragment nach einem der Ansprüche 18 bis 20 oder ein chimäres Gen nach einem der Ansprüche 21 bis 23 enthält.

31. Transformierte Pflanze, **dadurch gekennzeichnet, dass** sie mindestens eine pflanzliche transformierte Zelle nach Anspruch 30 umfasst.

32. Transformierte Pflanze nach Anspruch 31, **dadurch gekennzeichnet, dass** sie gegenüber Krankheiten verursacht durch *Cercospora,* insbesondere *Cercospora beticola, Cladosporium,* insbesondere *Cladosporium herbarum, Fusarium,* insbesondere *Fusarium culmorum* oder *Fusarium graminearum,* oder *Phytophthora,* insbesondere *Phytophthora cinnamomi,* resistent ist.

33. Transformierte Pflanze, **dadurch gekennzeichnet, dass** sie aus der Kultivierung und/oder der Kreuzung von Pflanzen nach einem der Ansprüche 31 oder 32 abgeleitet ist, und dass sie ein Nukleinsäurefragment nach einem der Ansprüche 18 bis 20 oder ein chimäres Gen nach einem der Ansprüche 21 bis 23 enthält.

34. Samen von transformierten Pflanzen nach einem der Ansprüche 31 bis 33, enthaltend ein Nukleinsäurefragment nach einem der Ansprüche 18 bis 20 oder ein chimäres Gen nach einem der Ansprüche 21 bis 23.

35. Verfahren zur Transformation von nicht-menschlichen Wirtsorganismen, insbesondere von pflanzlichen Zellen oder Pflanzen, **dadurch gekennzeichnet, dass** man in den besagten Wirtsorganis-mus mindestens ein Nukleinsäurefragment nach einem der Ansprüche 18 bis 20 oder ein chimäres Gen nach einem der Ansprüche 21 bis 23 einbringt.

36. Verfahren nach Anspruch 35, **dadurch gekennzeichnet, dass** der Wirtsorganismus eine pflanzliche Zelle oder eine Pflanze ist.

37. Verfahren nach Anspruch 36, **dadurch gekennzeichnet, dass** man eine Pflanze aus der pflanzlichen Zelle oder der transformierten Pflanze regeneriert.

38. Verfahren zur Kultivierung von transformierten Pflanzen nach einem der Ansprüche 31 bis 33, **dadurch gekennzeichnet, dass** es im Pflanzen der Samen der besagten transformierten Pflanzen in die Oberfläche eines geeigneten Feldes zur Kultivierung der besagten Pflanzen, im Anwenden einer agro-chemischen Zusammensetzung auf die besagte Oberfläche des besagten Feldes ohne die besagten Samen oder die besagten transformierten Pflanzen in einer wesentlichen Weise zu beeinflussen, dann im Gewinnen der kultivierten Pflanzen, sobald sie die gewünschte Reife erreicht haben, und gegebenenfalls im Abtrennen der Samen von den gewonnenen Pflanzen besteht.

39. Verfahren zur Kultivierung nach Anspruch 38, **dadurch gekennzeichnet, dass** die agrochemische Zusammensetzung mindestens ein wirksames Erzeugnis mit mindestens einer fungiziden und/oder bakteriziden Aktivität umfasst.

40. Verfahren zur Kultivierung nach Anspruch 39, **dadurch gekennzeichnet, dass** das wirksame Erzeugnis eine zu der des Peptids nach einem der Ansprüche 1 bis 15 komplementäre Aktivität zeigt.

41. Verfahren zur Herstellung von Heliomycin, definiert nach einem der Ansprüche 1 bis 15, **dadurch gekennzeichnet, dass** es die Schritte der Kultivierung eines transformierten Organismus nach einem der Ansprüche 25 bis 29 in einem geeigneten Kulturmedium, danach die Extraktion und die gesamte oder teilweise Reinigung des erhaltenen Heliomycins umfasst.


### LISTE DE SEQUENCES

(1) INFORMATIONS GENERALES:
   (i) DEPOSANT:
      (A) NOM: RHONE-POULENC AGROCHIMIE
      (B) RUE: 14-20 Rue Pierre BAIZET
      (C) VILLE: LYON
      (E) PAYS: France
      (F) CODE POSTAL: 69009
   (ii) TITRE DE L' INVENTION: Gène codant pour l'héliomicine, protéine obtenue, vecteur le contenant, organismes transformés obtenus et procédé de préparation
   (iii) NOMBRE DE SEQUENCES: 38
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: Patent In Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 147 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1. 147
   (xi) DESCRIPTION DE LA SEQUENCE: SEQ ID NO: 1:
(2) INFORMATIONS POUR LA SEQ ID NO: 2:
   (i) CARACTERISTIQUES DE LA SEQUENCE:
      (A) LONGUEUR: 169 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:1..132
   (xi) DESCRIPTION DE LA SEQUENCE: SEQ ID NO: 2:
   GAT AAG CTT ATC GGT TCC TGC GTG TGG GGT GCT GTG AAC TAC ACT TCC      48
   Asp Lys Leu Ile Gly Ser Cys Val Trp Gly Ala Val Asn Tyr Thr Ser
     1               5                  10                  15
   GAT TGC AAC GGT GAG TGC AAG AGG AGG GGT TAC AAG GGT GGT CAC TGC      96
   Asp Cys Asn Gly Glu Cys Lys Arg Arg Gly Tyr Lys Gly Gly His Cys
               20                  25                  30
   GGT TCC TTC GCT AAC GTG AAC TGC TGG TGC GAG ACT TGAGAGCTCG          142
   Gly Ser Phe Ala Asn Val Asn Cys Trp Cys Glu Thr
           35                  40
   GCGAGGCGAA CGTGTCGACG GATCCGG                                      169
(2) INFORMATIONS POUR LA SEQ ID NO: 3:
   (i) CARACTERISTIQUES DE LA SEQUENCE:
      (A) LONGUEUR: 261 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:3..224
   (xi) DESCRIPTION DE LA SEQUENCE: SEQ ID NO: 3:
   CC ATG GGT TTC GTG CTT TTC TCT CAG CTT CCA TCT TTC CTT CTT GTG          47
      Met Gly Phe Val Leu Phe Ser Gln Leu Pro Ser Phe Leu Leu Val
       1               5                  10                  15
   TCT ACT CTT CTT CTT TTC CTT GTG ATC TCT CAC TCT TGC CGT GCC GAT         95
   Ser Thr Leu Leu Leu Phe Leu Val Ile Ser His Ser Cys Arg Ala Asp
                   20                  25                  30
   AAG CTT ATC GGT TCC TGC GTG TGG GGT GCT GTG AAC TAC ACT TCC GAT        143
   Lys Leu Ile Gly Ser Cys Val Trp Gly Ala Val Asn Tyr Thr Ser Asp
               35                  40                  45
   TGC AAC GGT GAG TGC AAG AGG AGG GGT TAC AAG GGT GGT CAC TGC GGT        191
   Cys Asn Gly Glu Cys Lys Arg Arg Gly Tyr Lys Gly Gly His Cys Gly
           50                  55                  60
   TCC TTC GCT AAC GTG AAC TGC TGG TGC GAG ACT TGAGAGCTCG GCGAGGCGAA      244
   Ser Phe Ala Asn Val Asn Cys Trp Cys Glu Thr
       65                  70
   CGTGTCGACG GATCCGG                                                     261
(2) INFORMATIONS POUR LA SEQ ID NO: 4:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:12..101
   (xi) DESCRIPTION DE LA SEQUENCE: SEQ ID NO: 4:
(2) INFORMATIONS POUR LA SEQ ID NO: 5:
   (i) CARACTERISTIQUES DE LA SEQUENCE:
      (A) LONGUEUR: 75 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 7"
   (xi) DESCRIPTION DE LA SEQUENCE: SEQ ID NO: 5:
   GCGTCGACGC GATGGGTTTC GTGCTTTTCT CTCAGCTTCC ATCTTTCCTT CTTGTGTCTA 60
   CTCTTCTTCT TTTCC 75
(2) INFORMATIONS POUR LA SEQ ID NO: 6:
   (i) CARACTERISTIQUES DE LA SEQUENCE:
      (A) LONGUEUR: 72 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 8"
   (xi) DESCRIPTION DE LA SEQUENCE: SEQ ID NO: 6:
   TCGCCGGCAC GGCAAGAGTA AGAGATCACA AGGAAAAGAA GAAGAGTAGA CACAAGAAGG 60
   AAAGATGGAA GC 72
(2) INFORMATIONS POUR LA SEQ ID NO: 7:
   (i) CARACTERISTIQUES DE LA SEQUENCE:
      (A) LONGUEUR: 80 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléeotide synthétique 9"
   (xi) DESCRIPTION DE LA SEQUENCE: SEQ ID NO: 7:
   GATAAGCTTA TCGGTTCCTG CGTGTGGGGT GCTGTGAACT ACACTTCCGA TTGCAACGGT 60
   GAGTGCAAGA GGAGGGGTTA 80
(2) INFORMATIONS POUR LA SEQ ID NO: 8:
   (i) CARACTERISTIQUES DE LA SEQUENCE:
      (A) LONGUEUR: 109 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 10"
   (xi) DESCRIPTION DE LA SEQUENCE: SEQ ID NO: 8:
   CCGGATCCGT CGACACGTTC GCCTCGCCGA GCTCTCAAGT CTCGCACCAG CAGTTCACGT 60
   TAGCGAAGGA ACCGCAGTGA CCACCCTTGT AACCCCTCCT CTTGCACTC 109
(2) INFORMATIONS POUR LA SEQ ID NO: 9:
   (i) CARACTERISTIQUES DE LA SEQUENCE:
      (A) LONGUEUR: 85 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 11"
   (xi) DESCRIPTION DE LA SEQUENCE: SEQ ID NO: 9:
   AGGGCCCCCT AGGGTTTAAA CGGCCAGTCA GGCCGAATTC GAGCTCGGTA CCCGGGGATC 60
   CTCTAGAGTC GACCTGCAGG CATGC 85
(2) INFORMATIONS POUR LA SEQ ID NO: 10:
   (i) CARACTERISTIQUES DE LA SEQUENCE:
      (A) LONGUEUR: 66 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 12"
   (xi) DESCRIPTION DE LA SEQUENCE: SEQ ID NO: 10:
   CCCTGAACCA GGCTCGAGGG CGCGCCTTAA TTAAAAGCTT GCATGCCTGC AGGTCGACTC 60
   TAGAGG 66
(2) INFORMATIONS POUR LA SEQ ID NO: 11:
   (i) CARACTERISTIQUES DE LA SEQUENCE:
      (A) LONGUEUR: 93 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 13"
   (xi) DESCRIPTION DE LA SEQUENCE: SEQ ID NO: 11:
   CCGGCCAGTC AGGCCACACT TAATTAAGTT TAAACGCGGC CCCGGCGCGC CTAGGTGTGT 60
   GCTCGAGGGC CCAACCTCAG TACCTGGTTC AGG 93
(2) INFORMATIONS POUR LA SEQ ID NO: 12:
   (i) CARACTERISTIQUES DE LA SEQUENCE:
      (A) LONGUEUR: 93 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 14"
   (xi) DESCRIPTION DE LA SEQUENCE: SEQ ID NO: 12:
   CCGGCCTGAA CCAGGTACTG AGGTTGGGCC CTCGAGCACA CACCTAGGCG CGCCGGGGCC 60
   GCGTTTAAAC TTAATTAAGT GTGGCCTGAC TGG 93
(2) INFORMATIONS POUR LA SEQ ID NO: 13:
   (i) CARACTERISTIQUES DE LA SEQUENCE:
      (A) LONGUEUR: 50 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 15"
   (xi) DESCRIPTION DE LA SEQUENCE: SEQ ID NO: 13:
      GGTCTAGAAT GGCCTGCACC AACAACGCCA TGAGGGCCCT CTTCCTCCTC 50
(2) INFORMATIONS POUR LA SEQ ID NO: 14:
   (i) CARACTERISTIQUES DE LA SEQUENCE:
      (A) LONGUEUR: 50 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 16"
   (xi) DESCRIPTION DE LA SEQUENCE: SEQ ID NO: 14:
      CCGAATTCGG CGCCGTGCAC GATGCAGAAG AGCACGAGGA GGAAGAGGGC 50
(2) INFORMATIONS POUR LA SEQ ID NO: 15:
   (i) CARACTERISTIQUES DE LA SEQUENCE:
      (A) LONGUEUR: 81 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:7...73
   (xi) DESCRIPTION DE LA SEQUENCE SEQ ID NO:15:
   TCTAGA ATG GCC TGC ACC AAC AAC GCC ATG AGG GCC CTC TTC CTC CTC        48
          Met Ala Cys Thr Asn Asn Ala Met Arg Ala Leu Phe Cys Ile
          1               5                   10
   CTG CTC TTC TGC ATC GTG CAC GGC GCCGAATTC                             81
   Val Leu Phe Cys Ile Val His Gly
   15                  20
(2) INFORMATIONS POUR LA SEQ ID NO: 16:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 17"
   (xi) DESCRIPTION DE LA SEQUENCE: SEQ ID NO: 16:
      GATAAGCTTA TCGGTTCCTG CGTG 24
(2) INFORMATIONS POUR LA SEQ ID NO: 17:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 18"
   (xi) DESCRIPTION DE LA SEQUENCE: SEQ ID NO: 17:
      GGCTCGAGTC AAGTCTCGCA CCAGCAGTTC AC 32
2) INFORMATIONS POUR LA SEQ ID NO:18:
   (i) CARACTERISTIQUES DE LA SEQUENCE:
      (A) LONGUEUR: 213 paires de bases
      (B) TYPE: nucléotide
      (C) NOMBRE DE BRINS: simple
      (D) CONFIGURATION: linéaire
   (ii) TYPE DE MOLECULE: ADN
   (ix) CARACTERISTIQUE:
      (A) NOM/CLE: CDS
      (B) EMPLACEMENT:7...205
   (xi) DESCRIPTION DE LA SEQUENCE: SEQ ID NO:18:
   TCTAGA ATG GCC TGC ACC AAC AAC GCC ATG AGG GCC CTC TTC CTC CTC        48
          Met Ala Cys Thr Asn Asn Ala Met Arg Ala Leu Phe Cys Ile
          1               5                   10
   CTG CTC TTC TGC ATC GTG CAC GGC GAT AAG CTT ATC GGT TCC TGC GTG       96
   Val Leu Phe Cys Ile Val His Gly Asp Lys Leu Ile Gly Ser Cys Val
   15                  20                  25                  30
   TGG GGT GCT GTG AAC TAC ACT TCC GAT TGC AAC GGT GAG TGC AAG AGG      144
   Trp Gly Ala Val Asn Tyr Thr Ser Asp Cys Asn Gly Glu Cys Lys Arg
                   35                  40                  45
   AGG GGT TAC AAG GGT GGT CAC TGC GGT TCC TTC GCT AAC GTG AAC TGC      192
   Arg Gly Tyr Lys Gly Gly His Cys Gly Ser Phe Ala Asn Val Asn Cys
               50                  55                  60
   TGG TGC GAG ACT TGACTCGAG                                            213
   Trp Cys Glu Thr
           65
(2) INFORMATIONS POUR LA SEQ ID NO: 19:
   (i) CARACTERISTIQUES DE LA SEQUENCE:
      (A) LONGUEUR: 838 paires de bases
      (B) TYPE: nucléotide
      (C) NOMBRE DE BRINS: double
      (D) CONFIGURATION: linéaire
   (ii) TYPE DE MOLECULE: ADN
   (ix) CARACTERISTIQUE:
      (A) NOM/CLE: promoteur CsVMV
      (B) EMPLACEMENT:7..532
   (ix) CARACTERISTIQUE:
      (A) NOM/CLE: site de clonage multiple
      (B) EMPLACEMENT:533..568
   (ix) CARACTERISTIQUE:
      (A) NOM/CLE: terminateur
      (B) EMPLACEMENT:569..832
   (xi) DESCRIPTION DE LA SEQUENCE: SEQ ID NO: 19:
   AAGCTTCCAG AAGGTAATTA TCCAAGATGT AGCATCAAGA ATCCAATGTT TACGGGAAAA    60
   ACTATGGAAG TATTATGTGA GCTCAGCAAG AAGCAGATCA ATATGCGGCA CATATGCAAC   120
   CTATGTTCAA AAATGAAGAA TGTACAGATA CAAGATCCTA TACTGCCAGA ATACGAAGAA   180
   GAATACGTAG AAATTGAAAA AGAAGAACCA GGCGAAGAAA AGAATCTTGA AGACGTAAGC   240
   ACTGACGACA ACAATGAAAA GAAGAAGATA AGGTCGGTGA TTGTGAAAGA GACATAGAGG   300
   ACACATGTAA GGTGGAAAAT GTAAGGGCGG AAAGTAACCT TATCACAAAG GAATCTTATC   360
   CCCCACTACT TATCCTTTTA TATTTTTCCG TGTCATTTTT GCCCTTGAGT TTTCCTATAT   420
   AAGGAACCAA GTTCGGCATT TGTGAAAACA AGAAAAAATT TGGTGTAAGC TATTTTCTTT   480
   GAAGTACTGA GGATACAACT TCAGAGAAAT TTGTAAGTTT GTAGATCTCG ATTCTAGAAG   540
   GCCTGAATTC GAGCTCGGTA CCGGATCCAA TTCCCGATCG TTCAAACATT TGGCAATAAA   600
   GTTTCTTAAG ATTGAATCCT GTTGCCGGTC TTGCGATGAT TATCATATAA TTTCTGTTGA   660
   ATTACGTTAA GCATGTAATA ATTAACATGT AATGCATGAC GTTATTTATG AGATGGGTTT   720
   TTATGATTAG AGTCCCGCAA TTATACATTT AATACGCGAT AGAAAACAAA ATATAGCGCG   780
   CAAACTAGGA TAAATTATCG CGCGCGGTGT CATCTATGTT ACTAGATCGG GGATCGAT     838
(2) INFORMATIONS POUR LA SEQ ID NO: 20:
   (i) CARACTERISTIQUES DE LA SEQUENCE:
      (A) LONGUEUR: 1036 paires de bases
      (B) TYPE: nucléotide
      (C) NOMBRE DE BRINS: double
      (D) CONFIGURATION: linéaire
   (ii) TYPE DE MOLECULE: ADN
   (ix) CARACTERISTIQUE:
      (A) NOM/CLE: promoteur CsVMV
      (B) EMPLACEMENT:7..532
   (ix) CARACTERISTIQUE:
      (A) NOM/CLE: CDS
      (B) EMPLACEMENT:539..736
   (ix) CARACTERISTIQUE:
      (A) NOM/CLE: terminateur nos
      (B) EMPLACEMENT:767..1030
   (xi) DESCRIPTION DE LA SEQUENCE: SEQ ID NO: 20:
   AAGCTTCCAG AAGGTAATTA TCCAAGATGT AGCATCAAGA ATCCAATGTT TACGGGAAAA     60
   ACTATGGAAG TATTATGTGA GCTCAGCAAG AAGCAGATCA ATATGCGGCA CATATGCAAC    120
   CTATGTTCAA AAATGAAGAA TGTACAGATA CAAGATCCTA TACTGCCAGA ATACGAAGAA    180
   GAATACGTAG AAATTGAAAA AGAAGAACCA GGCGAAGAAA AGAATCTTGA AGACGTAAGC    240
   ACTGACGACA ACAATGAAAA GAAGAAGATA AGGTCGGTGA TTGTGAAAGA GACATAGAGG    300
   ACACATGTAA GGTGGAAAAT GTAAGGGCGG AAAGTAACCT TATCACAAAG GAATCTTATC    360
   CCCCACTACT TATCCTTTTA TATTTTTCCG TGTCATTTTT GCCCTTGAGT TTTCCTATAT    420
   AAGGAACCAA GTTCGGCATT TGTGAAAACA AGAAAAAATT TGGTGTAAGC TATTTTCTTT    480
   GAAGTACTGA GGATACAACT TCAGAGAAAT TTGTAAGTTT GTAGATCTCG ATTCTAGA      538
   ATG GCC TGC ACC AAC AAC GCC ATG AGG GCC CTC TTC CTC CTC GTG CTC      586
   Met Ala Cys Thr Asn Asn Ala Met Arg Ala Leu Phe Leu Leu Val Leu
    1               5                  10                  15
   TTC TGC ATC GTG CAC GGC GAT AAG CTT ATC GGT TCC TGC GTG TGG GGT      634
   Phe Cys Ile Val His Gly Asp Lys Leu Ile Gly Ser Cys Val Trp Gly
               20                  25                  30
   GCT GTG AAC TAC ACT TCC GAT TGC AAC GGT GAG TGC AAG AGG AGG GGT      682
   Ala Val Asn Tyr Thr Ser Asp Cys Asn Gly Glu Cys Lys Arg Arg Gly
           35                  40                  45
   TAC AAG GGT GGT CAC TGC GGT TCC TTC GCT AAC GTG AAC TGC TGG TGC      730
   Tyr Lys Gly Gly His Cys Gly Ser Phe Ala Asn Val Asn Cys Trp Cys
       50                  55                  60
   GAG ACT TGACTCGAGG GGGGGCCCGG TACCGGATCC AATTCCCGAT CGTTCAAACA      786
   Glu Thr
   65
   TTTGGCAATA AAGTTTCTTA AGATTGAATC CTGTTGCCGG TCTTGCGATG ATTATCATAT    846
   AATTTCTGTT GAATTACGTT AAGCATGTAA TAATTAACAT GTAATGCATG ACGTTATTTA    906
   TGAGATGGGT TTTTATGATT AGAGTCCCGC AATTATACAT TTAATACGCG ATAGAAAACA    966
   AAATATAGCG CGCAAACTAG GATAAATTAT CGCGCGCGGT GTCATCTATG TTACTAGATC   1026
   GGGGATCGAT                                                          1036
(2) INFORMATIONS POUR LA SEQ ID NO: 21:
   (i) CARACTERISTIQUES DE LA SEQUENCE:
      (A) LONGUEUR : 52 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 1"
   (xi) DESCRIPTION DE LA SEQUENCE: SEQ ID NO: 21:
      AGCTTGGATA AAAGAGACAA GTTGATTGGC AGCTGTGTTT GGGGCGCCGT CA 52
(2) INFORMATIONS POUR LA SEQ ID NO: 22:
   (i) CARACTERISTIQUES DE LA SEQUENCE:
      (A) LONGUEUR: 56 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 2"
   (xi) DESCRIPTION DE LA SEQUENCE: SEQ ID NO: 22:
      AGTGTAGTTG ACGGCGCCCC AAACACAGCT GCCAATCAAC TTGTCTCTTT TATCCA 56
(2) INFORMATIONS POUR LA SEQ ID NO: 23:
   (i) CARACTERISTIQUES DE LA SEQUENCE:
      (A) LONGUEUR: 52 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 3"
   (xi) DESCRIPTION DE LA SEQUENCE: SEQ ID NO: 23:
      ACTACACTAG TGACTGCAAC GGCGAGTGCA AGCGCCGCGG TTACAAGGGT GG 52
(2) INFORMATIONS POUR LA SEQ ID NO: 24:
   (i) CARACTERISTIQUES DE LA SEQUENCE:
      (A) LONGUEUR: 52 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 4"
   (xi) DESCRIPTION DE LA SEQUENCE: SEQ ID NO: 24:
      CACAATGGCC ACCCTTGTAA CCGCGGCGCT TGCACTCGCC GTTGCAGTCA CT 52
(2) INFORMATIONS POUR LA SEQ ID NO: 25:
   (i) CARACTERISTIQUES DE LA SEQUENCE:
      (A) LONGUEUR: 56 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 5"
   (xi) DESCRIPTION DE LA SEQUENCE: SEQ ID NO: 25:
      CCATTGTGGA TCCTTCGCTA ACGTTAACTG TTGGTGTGAA ACCTGATAGG TCGACA 56
(2) INFORMATIONS POUR LA SEQ ID NO: 26:
   (i) CARACTERISTIQUES DE LA SEQUENCE
      (A) LONGUEUR: 52 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 6"
   (xi) DESCRIPTION DE LA SEQUENCE: SEQ ID NO: 26:
      GATCTGTCGA CCTATCAGGT TTCACACCAA CAGTTAACGT TAGCGAAGGA TC 52
(2) INFORMATIONS POUR LA SEQ ID NO: 27:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 19"
   (xi) DESCRIPTION DE LA SEQUENCE: SEQ ID NO: 27:
      GATCCTTCGC TAACGTTAAC TGTTGGTGTA GAACCTGATA GG 42
(2) INFORMATIONS POUR LA SEQ ID NO: 28:
   (i) CARACTERISTIQUES DE LA SEQUENCE:
      (A) LONGUEUR: 42 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 20"
   (xi) DESCRIPTION DE LA SEQUENCE: SEQ ID NO: 28:
      TCGACCTATC AGGTTCTACA CCAACAGTTA ACGTTAGCGA AG 42
(2) INFORMATIONS POUR LA SEQ ID NO: 29:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 21"
   (xi) DESCRIPTION DE LA SEQUENCE: SEQ ID NO: 29:
      CTAGTGACTG CAACGGCGAG TGCTTGTTGC GC 32
(2) INFORMATIONS POUR LA SEQ ID NO: 30:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 22"
   (xi) DESCRIPTION DE LA SEQUENCE: SEQ ID NO: 30:
      GCAACAAGCA CTCGCCGTTG CAGTCA 26
(2) INFORMATIONS POUR LA SEQ ID NO: 31:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 23"
   (xi) DESCRIPTION DE LA SEQUENCE: SEQ ID NO: 31:
      CTAGTGACTG CGCTGCTGAG TGCAAGCGGC GC 32
(2) INFORMATIONS POUR LA SEQ ID NO: 32:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 24"
   (xi) DESCRIPTION DE LA SEQUENCE: SEQ ID NO: 32:
      GCCGCTTGCA CTCAGCAGCG CAGTCA 26
(2) INFORMATIONS POUR LA SEQ ID NO: 33:
   (i) CARACTERISTIQUES DE LA SEQUENCE:
      (A) LONGUEUR: 40 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 25"
   (xi) DESCRIPTION DE LA SEQUENCE: SEQ ID NO: 33:
      AGCTTGGATA AAAGAGCTGC TGCTGCTGGT AGCTGTGTTT 40
(2) INFORMATIONS POUR LA SEQ ID NO: 34:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 26"
   (xi) DESCRIPTION DE LA SEQUENCE: SEQ ID NO: 34:
      GGGGCGCCGT CAACTACA 18
(2) INFORMATIONS POUR LA SEQ ID NO: 35:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 27"
   (xi) DESCRIPTION DE LA SEQUENCE: SEQ ID NO: 35:
      CTAGTGTAGT TGACGGCGCC CC 22
(2) INFORMATIONS POUR LA SEQ ID NO: 36:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 28"
   (xi) DESCRIPTION DE LA SEQUENCE: SEQ ID NO: 36:
      AAACACAGCT ACCAGCAGCA GCAGCTCTTT TATCCA 36
(2) INFORMATIONS POUR LA SEQ ID NO: 37:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 29"
   (xi) DESCRIPTION DE LA SEQUENCE: SEQ ID NO: 37:
      CTAGTGACTG CGCTGCTGAG TGCTTGTTGC GC 32
(2) INFORMATIONS POUR LA SEQ ID NO: 38:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucléotide synthétique 30"
   (xi) DESCRIPTION DE LA SEQUENCE: SEQ ID NO: 38:
      GCAACAAGCA CTCAGCAGCG CAGTCA 26

### LISTE DE SEQUENCES

<110> RHONE-POULENC AGRO
<120> GENE CODANT POUR L'HELIOMICINE, PROTEINE OBTENUE, VECTEUR LE CONTENANT, ORGANISMES TRANSFORMES OBTENUS ET PROCEDE DE PREPARATION
<140> PCT/FR99/00843
   <141> 1999-04-12
<150> FR 98 04933
   <151> 1998-04-15
<160> 38
<170> PatentIn Ver. 2.1
<210> 1
   <211> 147
   <212> ADN
   <213> synthetic construct
<220>
   <221> CDS
   <222> (1)..(147)
<400> 1
   agc ttg gat aaa aga gac aag ttg att ggc agc tgt gtt tgg ggc gcc     48
   Ser Leu Asp Lys Arg Asp Lys Leu Ile Gly Ser Cys Val Trp Gly Ala
    1               5                  10                  15
   gtc aac tac act agt gac tgc aac ggc gag tgc aag cgc cgc ggt tac     96
   Val Asn Tyr Thr Ser Asp Cys Asn Gly Glu Cys Lys Arg Arg Gly Tyr
               20                  25                  30
   aag ggt ggc cat tgt gga tcc ttc gct aac gtt aac tgt tgg tgt gaa    144
   Lys Gly Gly His Cys Gly Ser Phe Ala Asn Val Asn Cys Trp Cys Glu
           35                  40                  45
   acc                                                                147
   Thr
<210> 2
   <211> 169
   <212> ADN
   <213> synthetic construct
<220>
   <221> CDS
   <222> (1)..(132)
<400> 2
   gat aag ctt atc ggt tcc tgc gtg tgg ggt gct gtg aac tac act tcc     48
   Asp Lys Leu Ile Gly Ser Cys Val Trp Gly Ala Val Asn Tyr Thr Ser
    1               5                  10                  15
   gat tgc aac ggt gag tgc aag agg agg ggt tac aag ggt ggt cac tgc     96
   Asp Cys Asn Gly Glu Cys Lys Arg Arg Gly Tyr Lys Gly Gly His Cys
               20                  25                  30
   ggt tcc ttc gct aac gtg aac tgc tgg tgc gag act tgagagctcg         142
   Gly Ser Phe Ala Asn Val Asn Cys Trp Cys Glu Thr
           35                  40
   gcgaggcgaa cgtgtcgacg gatccgg                                      169
<210> 3
   <211> 261
   <212> ADN
   <213> synthetic construct
<220>
   <221> CDS
   <222> (3) . . (224)
<400> 3
   cc atg ggt ttc gtg ctt ttc tct cag ctt cca tct ttc ctt ctt gtg    47
      Met Gly Phe Val Leu Phe Ser Gln Leu Pro Ser Phe Leu Leu Val
       1               5                   10                  15
   tct act ctt ctt ctt ttc ctt gtg atc tct cac tct tgc cgt gcc gat   95
   Ser Thr Leu Leu Leu Phe Leu Val Ile Ser His Ser Cys Arg Ala Asp
                   20                  25                  30
   aag ctt atc ggt tcc tgc gtg tgg ggt gct gtg aac tac act tcc gat   143
   Lys Leu Ile Gly Ser Cys Val Trp Gly Ala Val Asn Tyr Thr Ser Asp
               35                  40                  45
   tgc aac ggt gag tgc aag agg agg ggt tac aag ggt ggt cac tgc ggt   191
   Cys Asn Gly Glu Cys Lys Arg Arg Gly Tyr Lys Gly Gly His Cys Gly
           50                  55                  60
   tcc ttc gct aac gtg aac tgc tgg tgc gag act tgagagctcg gcgaggcgaa 244
   Ser Phe Ala Asn Val Asn Cys Trp Cys Glu Thr
       65                  70
   cgtgtcgacg gatccgg                                                261
<210> 4
   <211> 120
   <212> ADN
   <213> synthetic construct
<220>
   <221> CDS
   <222> (12)..(101)
<400> 4
   gcgtcgacgc g atg ggt ttc gtg ctt ttc tct cag ctt cca tct ttc ctt  50
                Met Gly Phe Val Leu Phe Ser Gln Leu Pro Ser Phe Leu
                 1               5                   10
   ctt gtg tct act ctt ctt ctt ttc ctt gtg atc tct cac tct tgc cgt   98
   Leu Val Ser Thr Leu Leu Leu Phe Leu Val Ile Ser His Ser Cys Arg
       15                  20                  25
   gct ggagacgcga attcacaca                                          120
   Ala
    30
<210> 5
   <211> 75
   <212> ADN
   <213> synthetic construct
<400> 5
   gcgtcgacgc gatgggtttc gtgcttttct ctcagcttcc atctttcctt cttgtgtcta 60
   ctcttcttct tttcc 75
<210> 6
   <211> 72
   <212> ADN
   <213> synthetic construct
<400> 6
   tcgccggcac ggcaagagta agagatcaca aggaaaagaa gaagagtaga cacaagaagg 60
   aaagatggaa gc 72
<210> 7
   <211> 80
   <212> ADN
   <213> synthetic construct
<400> 7
   gataagctta tcggttcctg cgtgtggggt gctgtgaact acacttccga ttgcaacggt 60
   gagtgcaaga ggaggggtta 80
<210> 8
   <211> 109
   <212> ADN
   <213> synthetic construct
<400> 8
   ccggatccgt cgacacgttc gcctcgccga gctctcaagt ctcgcaccag cagttcacgt 60
   tagcgaagga accgcagtga ccacccttgt aacccctcct cttgcactc 109
<210> 9
   <211> 85
   <212> ADN
   <213> synthetic construct
<400> 9
   agggccccct agggtttaaa cggccagtca ggccgaattc gagctcggta cccggggatc 60
   ctctagagtc gacctgcagg catgc 85
<210> 10
   <211> 66
   <212> ADN
   <213> synthetic construct
<400> 10
   ccctgaacca ggctcgaggg cgcgccttaa ttaaaagctt gcatgcctgc aggtcgactc 60
   tagagg 66
<210> 11
   <211> 93
   <212> ADN
   <213> synthetic construct
<400> 11
   ccggccagtc aggccacact taattaagtt taaacgcggc cccggcgcgc ctaggtgtgt 60
   gctcgagggc ccaacctcag tacctggttc agg 93
<210> 12
   <211> 93
   <212> ADN
   <213> synthetic construct
<400> 12
   ccggcctgaa ccaggtactg aggttgggcc ctcgagcaca cacctaggcg cgccggggcc 60
   gcgtttaaac ttaattaagt gtggcctgac tgg 93
<210> 13
   <211> 50
   <212> ADN
   <213> synthetic construct
<400> 13
   ggtctagaat ggcctgcacc aacaacgcca tgagggccct cttcctcctc 50
<210> 14
   <211> 50
   <212> ADN
   <213> synthetic construct
<400> 14
   ccgaattcgg cgccgtgcac gatgcagaag agcacgagga ggaagagggc 50
<210> 15
   <211> 81
   <212> ADN
   <213> synthetic construct
<220>
   <221> CDS
   <222> (7)..(72)
<400> 15
   tctaga atg gcc tgc acc aac aac gcc atg agg gcc ctc ttc ctc ctc     48
          Met Ala Cys Thr Asn Asn Ala Met Arg Ala Leu Phe Leu Leu
            1               5                   10
   ctg ctc ttc tgc atc gtg cac ggc gccgaattc                           81
   Leu Leu Phe Cys Ile Val His Gly
    15                  20
<210> 16
   <211> 24
   <212> ADN
   <213> synthetic construct
<400> 16 24
   gataagctta tcggttcctg cgtg 24
<210> 17
   <211> 32
   <212> ADN
   <213> synthetic construct
<400> 17
   ggctcgagtc aagtctcgca ccagcagttc ac 32
<210> 18
   <211> 213
   <212> ADN
   <213> synthetic construct
<220>
   <221> CDS
   <222> (7)..(204)
<400> 18
   tctaga atg gcc tgc acc aac aac gcc atg agg gcc ctc ttc ctc ctc     48
          Met Ala Cys Thr Asn Asn Ala Met Arg Ala Leu Phe Leu Leu
            1               5                   10
   ctg ctc ttc tgc atc gtg cac ggc gat aag ctt atc ggt tcc tgc gtg     96
   Leu Leu Phe Cys Ile Val His Gly Asp Lys Leu Ile Gly Ser Cys Val
    15                  20                  25                  30
   tgg ggt gct gtg aac tac act tcc gat tgc aac ggt gag tgc aag agg    144
   Trp Gly Ala Val Asn Tyr Thr Ser Asp Cys Asn Gly Glu Cys Lys Arg
                    35                  40                  45
   agg ggt tac aag ggt ggt cac tgc ggt tcc ttc gct aac gtg aac tgc    192
   Arg Gly Tyr Lys Gly Gly His Cys Gly Ser Phe Ala Asn Val Asn Cys
                50                  55                  60
   tgg tgc gag act tgactcgag                                          213
   Trp Cys Glu Thr
            65
<210> 19
   <211> 838
   <212> ADN
   <213> synthetic construct
<220>
   <221> promoter
   <222> (7)..(532)
<220>
   <221> misc_structure
   <222> (533)..(568)
<220>
   <221> terminator
   <222> (569)..(832)
<400> 19
   aagcttccag aaggtaatta tccaagatgt agcatcaaga atccaatgtt tacgggaaaa 60
   actatggaag tattatgtga gctcagcaag aagcagatca atatgcggca catatgcaac 120
   ctatgttcaa aaatgaagaa tgtacagata caagatccta tactgccaga atacgaagaa 180
   gaatacgtag aaattgaaaa agaagaacca ggcgaagaaa agaatcttga agacgtaagc 240
   actgacgaca acaatgaaaa gaagaagata aggtcggtga ttgtgaaaga gacatagagg 300
   acacatgtaa ggtggaaaat gtaagggcgg aaagtaacct tatcacaaag gaatcttatc 360
   ccccactact tatcctttta tatttttccg tgtcattttt gcccttgagt tttcctatat 420
   aaggaaccaa gttcggcatt tgtgaaaaca agaaaaaatt tggtgtaagc tattttcttt 480
   gaagtactga ggatacaact tcagagaaat ttgtaagttt gtagatctcg attctagaag 540
   gcctgaattc gagctcggta ccggatccaa ttcccgatcg ttcaaacatt tggcaataaa 600
   gtttcttaag attgaatcct gttgccggtc ttgcgatgat tatcatataa tttctgttga 660
   attacgttaa gcatgtaata attaacatgt aatgcatgac gttatttatg agatgggttt 720
   ttatgattag agtcccgcaa ttatacattt aatacgcgat agaaaacaaa atatagcgcg 780
   caaactagga taaattatcg cgcgcggtgt catctatgtt actagatcgg ggatcgat   838
<210> 20
   <211> 1036
   <212> ADN
   <213> synthetic construct
<220>
   <221> promoter
   <222> (7)..(532)
<220>
   <221> CDS
   <222> (539)..(736)
<220>
   <221> terminator
   <222> (767)..(1030)
<400> 20
   aagcttccag aaggtaatta tccaagatgt agcatcaaga atccaatgtt tacgggaaaa 60
   actatggaag tattatgtga gctcagcaag aagcagatca atatgcggca catatgcaac 120
   ctatgttcaa aaatgaagaa tgtacagata caagatccta tactgccaga atacgaagaa 180
   gaatacgtag aaattgaaaa agaagaacca ggcgaagaaa agaatcttga agacgtaagc 240
   actgacgaca acaatgaaaa gaagaagata aggtcggtga ttgtgaaaga gacatagagg 300
   acacatgtaa ggtggaaaat gtaagggcgg aaagtaacct tatcacaaag gaatcttatc 360
   ccccactact tatcctttta tatttttccg tgtcattttt gcccttgagt tttcctatat 420
   aaggaaccaa gttcggcatt tgtgaaaaca agaaaaaatt tggtgtaagc tattttcttt 480
   gaagtactga ggatacaact tcagagaaat ttgtaagttt gtagatctcg attctaga   538
   atg gcc tgc acc aac aac gcc atg agg gcc ctc ttc ctc ctc gtg ctc   586
   Met Ala Cys Thr Asn Asn Ala Met Arg Ala Leu Phe Leu Leu Val Leu
    1               5                   10                  15
   ttc tgc atc gtg cac ggc gat aag ctt atc ggt tcc tgc gtg tgg ggt   634
   Phe Cys Ile Val His Gly Asp Lys Leu Ile Gly Ser Cys Val Trp Gly
               20                  25                  30
   gct gtg aac tac act tcc gat tgc aac ggt gag tgc aag agg agg ggt   682
   Ala Val Asn Tyr Thr Ser Asp Cys Asn Gly Glu Cys Lys Arg Arg Gly
           35                  40                  45
   tac aag ggt ggt cac tgc ggt tcc ttc gct aac gtg aac tgc tgg tgc   730
   Tyr Lys Gly Gly His Cys Gly Ser Phe Ala Asn Val Asn Cys Trp Cys
       50                  55                  60
   gag act tgactcgagg gggggcccgg taccggatcc aattcccgat cgttcaaaca    786
   Glu Thr
    65
   tttggcaata aagtttctta agattgaatc ctgttgccgg tcttgcgatg attatcatat 846
   aatttctgtt gaattacgtt aagcatgtaa taattaacat gtaatgcatg acgttattta 906
   tgagatgggt ttttatgatt agagtcccgc aattatacat ttaatacgcg atagaaaaca 966
   aaatatagcg cgcaaactag gataaattat cgcgcgcggt gtcatctatg ttactagatc 1026
   ggggatcgat                                                        1036
<210> 21
   <211> 52
   <212> ADN
   <213> synthetic construct
<400> 21
   agcttggata aaagagacaa gttgattggc agctgtgttt ggggcgccgt ca 52
<210> 22
   <211> 56
   <212> ADN
   <213> synthetic construct
<400> 22
   agtgtagttg acggcgcccc aaacacagct gccaatcaac ttgtctcttt tatcca 56
<210> 23
   <211> 52
   <212> ADN
   <213> synthetic construct
<400> 23
   actacactag tgactgcaac ggcgagtgca agcgccgcgg ttacaagggt gg 52
<210> 24
   <211> 52
   <212> ADN
   <213> synthetic construct
<400> 24
   cacaatggcc acccttgtaa ccgcggcgct tgcactcgcc gttgcagtca ct 52
<210> 25
   <211> 56
   <212> ADN
   <213> synthetic construct
<400> 25
   ccattgtgga tccttcgcta acgttaactg ttggtgtgaa acctgatagg tcgaca 56
<210> 26
   <211> 52
   <212> ADN
   <213> synthetic construct
<400> 26
   gatctgrcga cctatcaggt ttcacaccaa cagttaacgt tagcgaagga tc 52
<210> 27
   <211> 42
   <212> ADN
   <213> synthetic construct
<400> 27
   gatccttcgc taacgttaac tgttggtgta gaacctgata gg 42
<210> 28
   <211> 42
   <212> ADN
   <213> synthetic construct
<400> 28
   tcgacctatc aggttctaca ccaacagtta acgttagcga ag 42
<210> 29
   <211> 32
   <212> ADN
   <213> synthetic construct
<400> 29
   ctagtgactg caacggcgag tgcttgttgc gc 32
<210> 30
   <211> 26
   <212> ADN
   <213> synthetic construct
<400> 30
   gcaacaagca ctcgccgttg cagtca 26
<210> 31
   <211> 32
   <212> ADN
   <213> synthetic construct
<400> 31
   ctagtgactg cgctgctgag tgcaagcggc gc 32
<210> 32
   <211> 26
   <212> ADN
   <213> synthetic construct
<400> 32
   gccgcttgca ctcagcagcg cagtca 26
<210> 33
   <211> 40
   <212> ADN
   <213> synthetic construct
<400> 33
   agcttggata aaagagctgc tgctgctggt agctgtgttt 40
<210> 34
   <211> 18
   <212> ADN
   <213> synthetic construct
<400> 34
   ggggcgccgt caactaca 18
<210> 35
   <211> 22
   <212> ADN
   <213> synthetic construct
<400> 35
   ctagtgtagt tgacggcgcc cc 22
<210> 36
   <211> 36
   <212> ADN
   <213> synthetic construct
<400> 36
   aaacacagct accagcagca gcagctcttt tatcca 36
<210> 37
   <211> 32
   <212> ADN
   <213> synthetic construct
<400> 37
   ctagtgactg cgctgctgag tgcttgttgc gc 32
<210> 38
   <211> 26
   <212> ADN
   <213> synthetic construct
<400> 38
   gcaacaagca ctcagcagcg cagtca 26